# EUROPEAN PATENT APPLICATION

(11) **EP 1 627 926 A1**
(43) Date of publication of application: **22.02.2006**
(21) Application number: 05017712.0
(22) Date of filing: 16.08.2005
(51) Int. Cl.: C12Q 1/68

(54) **A method to reduce false positive results**

(30) Priority: 19.08.2004 EP 04019636
(71) Applicant: Roche Diagnostics GmbH, 68305 Mannheim (DE); F.HOFFMANN-LA ROCHE AG, 4070 Basel (CH)
(72) Inventor: Bergmann, Frank, 82393 Iffeldorf (DE); Escherich, Achim, 82439 Grossweil (DE); Heindl, Dieter, 82396 Paehl (DE); Krebs, Jane, 82319 Starnberg (DE); von der Eltz, Herbert, 82362 Weilheim (DE)

(57) **Abstract**

The present invention provides a method and a compound for the decontamination of liquids from certain molecules. In particular, the present invention is directed to a method and a compound that maintains reagents free from double-stranded nucleic acid molecules. More particular, the present invention is directed to a method and a compound that ensures a nucleic acid amplification without the amplification of contaminations.

SEQUENCE LISTING

<110>  Hoffmann La Roche AG

<120>  A method to reduce false positive results

<130>  22697FF

<150>  EP 04019636
<151>  2004-08-19

<160>  1

<170>  PatentIn version 3.2

<210>  1
<211>  25
<212>  DNA
<213>  Artificial

<220>
<223>  Artifical sequence for example


<220>
<221>  3'-phosphate
<222>  (25)..(25)

<400>  1
cccatcccca aaaacacaaa ccaca                                          25

## Description

### Field of invention

The present invention provides a method and a compound for the decontamination of liquids from certain molecules. In particular, the present invention is directed to a method and a compound that maintains reagents free from said molecules. More particular, the present invention is directed to a method and a compound that ensures a nucleic acid amplification reaction of a target molecule in a sample avoiding false positive results.

### Prior art background

Microbiological tests for diagnostics or research based on nucleic acid analysis are of still increasing importance. Since on the one hand, the nucleic acids are often present in very small concentrations and, on the other hand, they are often found in the presence of many other solid and dissolved substances e.g. after lysis of cells, they are difficult to isolate or to measure, in particular in biospecific assays which allow the detection of specific analytes. Therefore, in the majority of cases, these microbiological tests comprise at least one amplification step of the characteristic DNA molecules to be detected. A well-known assay which entails the selective binding of two oligonucleotide primers is the polymerase chain reaction (PCR) described in US 4,683,195. This method allows the selective amplification of a specific nucleic acid region to detectable levels by a thermostable polymerase in the presence of deoxynucleotide triphosphates in several cycles. Other possible amplification reactions are the Ligase Chain Reaction (LCR, Wu, D.Y. and Wallace, R.B., Genomics 4 (1989) 560-569 and Barany, Proc. Natl. Acad. Sci. USA 88 (1991) 189-193); Polymerase Ligase Chain Reaction (Barany, PCR Methods and Applic. 1 (1991) 5-16); Gap-LCR (PCT Patent Publication No. WO 90/01069); Repair Chain Reaction (European Patent Publication No. 439 182 A2), 3SR (Kwoh, D.Y. et al., Proc. Natl. Acad. Sci. USA 86 (1989) 1173-1177; Guatelli, J.C. et al., Proc. Natl. Acad. Sci. USA 87 (1990) 1874-1878; PCT Patent Publication No. WO 92/0880A), and NASBA (U.S. Pat. No. 5,130,238). Further, there are strand displacement amplification (SDA), transciption mediated amplification (TMA), and Qβ-amplification (for a review see e.g. Whelen, A.C. and Persing,D.H., Annu. Rev. Microbiol. 50 (1996) 349-373; Abramson, R.D. and Myers, T.W., Current Opinion in Biotechnology 4 (1993) 41-47).

Since these techniques result in a drastic amplification of nucleic acid molecules, even the slightest contamination of the sample with undesired nucleic acid molecules from the reagents necessary for said amplification may result in a huge amount of false amplification products synonymous to e.g. a false positive diagnosis. A prominent example of such a phenomenon may occur in a microbiological test, where the existence of a bacterium in a sample should be tested and some of the used reagents are contaminated with this bacterium or with nucleic acids derived from this bacterium as well. In this case, the target nucleic acid molecules from the sample under investigation are identical to the undesired molecules from the contaminated reagent.

Therefore, the requirements towards contamination-free environments of nucleic acid amplification reactions are of utmost importance.

In the scientific literature and in patent applications several procedures for the decontamination of solutions from nucleic acids are described. These procedures include chemical treatments with e.g. Clorox (Prince, A.M. and Andrus, L., Biotechniques 12 (1992) 358-360), surfactants or oxidizing agents (WO 2002/060539). Other strategies utilize treatments with digestion enzymes (EP 0 585 660; Hilali, F. et al, Mol. Biotechnol. 7 (1997) 207-216) or UV illumination (Fox, J.C. et al, J. Virol. Meth. 33 (1991) 375-3823). Concerning the above mentioned strategies several review articles can be found in literature (Abravaya, K. et al, Nucleic Acid Ampl. Technol. Chapter 9 (1997) 125-133; Corless, C.E. et al, J. Clin. Microbiol. 38 (2000) 1747-1752; Klaschik, S. et al, Mol. Biotechnol. 22 (2002) 231-242).

An other approach, normally applied for the separation or isolation of e.g. DNA from complex biological fluids, is the use of DNA binding materials. The most prominent example of DNA binding material are glass surfaces due to their ability to reversibly bind DNA in the presence of chaotropic reagents (Vogelstein, B., and Gillespie, D., Proc. Natl. Acad. Sci. USA 76 (1979) 615-619). However, this process does not distinguish between single- and double-stranded DNA. US 04/121336 descibes a method of binding nucleic acid to a multiplicity of solid substrate binding units.
More general, cationic surfaces may be used to bind charged DNA molecules, whereby e.g. EP 0 281390 describes a polycationic support for nucleic acid isolation, WO 01/94573 charged membranes or WO 00/69872 a pH dependent ion exchange matrix. WO 02/48164 discloses polymers with switchable charge on solid supports for reversible binding of DNA. Similar to cationic surfaces, polycationic entities have certain DNA-binding affinity, too. Stewart, et al., J. Phys. Org. Chem. 5 (1992) 461-466 reports an increasing affinity of polyamines in solution for binding to DNA with increasing cationic charge. Doré et al JACS 126 (2004) 4240-4244) describes the selectivity of cationic compounds between double-stranded and single-stranded nucleic acids.

Moreover, double-stranded (ds) DNA binding molecules are known in the art that may distinguish between single- and double-stranded DNA. Here, one should mention especially minor groove binders (MGBs), anti-ds DNA antibodies and intercalators.

MGBs like the pyrrol amidine antibiotics Distamycin or Netropsin bind to the minor groove by hydrogen bonding and do not interact by intercalation (Fish, E.L. et al, Biochemistry 27 (1988) 6026-6032). Boger, D.L. et al., J. Am. Chem. Soc. 123 (2001) 5878-5891 studied the binding of Distamycin, Netropsin and 4',6-diamidino-2-phenylindole (DAPI) to double-stranded DNA immobilized to microtiter plates. US 2002/0095073 describes the use of MGBs as probes that are immobilized on a solid support via amide or thiol bonds to bind target DNA in order to determine a cause of one or more medical symptoms. Li, M. et al Bioorg. Med. Chem. Letters 12 (2003) 4351-4354 describes a DAPI derivative that may bind by a covalent amide bond to amino groups of the surface.

Anti-ds DNA antibodies are known to have the ability to distinguish between single- and double-standed DNA and therefore, they were used e.g. as a probe for DNA hybrids immobilized to a solid support, whereby the presence of the antibody was verified by colorimetric detection (Mantero, G. et al, Clin. Chem. 37 (1991) 422-429). WO 2002/074993 describes a method to immobilize an anti-DNA antibody on gold covered glass slides using an activated thiol monolayer. The patent EP 0 792 376 relates to the field of nucleic acid amplification reactions and discloses the immobilization of anti-hybrid antibodies to surfaces via a biotin/streptavidin bond.

In the state of the art immobilized antibodies are also used to reversibly bind cells or assemblies of cells, e.g. bacteria. For the separation of food pathogens there are already commercial products based on beads functionalized with antibodies (DYNAL Biotech or MATRIX Microscience Ltd). The WO 91/19003 discloses a process to detect contaminations using antibodies on surfaces. PROFOS GmbH discloses the use of bacteriophage tail proteins coupled to a carrier to bind bacteria (WO 01/09370, WO 02/06117) or endotoxins (WO 04/01418). Koepsel and Russell (Koepsel, R.R. and Russell, A.J., Biomacromolecules 4 (2003) 850-855) disclose antibodies on bioplastic films to capture bacteria. For an unspecific accumulation of bacteria, the WO 03/33698 describes a method with charged polymers.

The intercalating properties of some small organic molecules that bind between stacked base pairs of double stranded DNA was intensively studied in the past (Brana, M.F. et al, Curr. Pharm. Design 7 (2001) 1745-1780). WO 2002/82078 describes complexes out of intercalators and signaling molecules in order to detect the presence of double-stranded DNA on a solid support. The use of intercalators attached to a support to separate single- and double-stranded DNA is described in US 2002/0006617. US 2001/0026921 discloses a nucleic acid hybridization assay composition with intercalators bound to a surface via an amino functionality and a second intercalator bearing a fluorophore.

US 2003/0130499 discloses the use of e.g. Actinomycin D or ethidium bromide as intercalators that may be incorporated into a solid phase in order to bind nucleic acids.

### Brief description of the invention:

Many alternatives for the binding of biological molecules, like e.g. nucleic acids, from a liquid solution to the surface of a solid support are known to someone skilled in the art, but non of these alternatives are applicable as a decontamination method for special applications.

Therefore, the present invention is directed to a method and a compound for the detection of biological molecules in a sample that decontaminates reagents, necessary for said detection, from and/or maintains said reagents free of biological molecules, whereby the decontamination process is permanently active in the background of a certain experiment. More particular, the present invention is directed to an improved method and a compound that ensures a nucleic acid amplification performance without the amplification of non-target nucleic acid molecules and/or target nucleic acid molecules potentially present in said reagents via a nucleic acid binding compound that is permanently present until the actual nucleic acid amplification.

One subject matter of the present invention is a method for the detection of biological molecules in a sample that avoids the detection of biological molecules potentially present in the reagents necessary to detect the biological molecules in the sample, comprising the steps of
a) providing a sample potentially comprising said biological molecule,
b) providing binding moieties coupled to the surface of a solid phase,
c) providing reagents necessary to detect the biological molecules,
d) adding said reagents to said sample,
e) detecting the biological molecules in said sample and
wherein in step c) or in step d) or in step c) and d) said reagents are in physical contact with said binding moieties under conditions, whereby said biological molecules potentially present in said reagents bind to said binding moieties coupled to said surface of a solid phase.

The invention also concerns a method to amplify a target nucleic acid molecule comprising the steps of
a) providing a sample potentially comprising target nucleic acid molecules,
b) providing nucleic acid binding moieties coupled to the surface of a solid phase,
c) providing reagents necessary to amplify said target nucleic acid molecules,
d) adding said reagents to said sample
e) amplifying said target nucleic acid molecules in said sample and
wherein in step c) or in step d) or in step c) and d) said reagents are in physical contact with said nucleic acid binding moieties under conditions, whereby said nucleic acid molecules potentially present in said reagents bind to said nucleic acid binding moieties coupled to said surface of a solid phase.

In alternative embodiments of the invention, the steps a) to c) are performed in any other possible succession of these steps.

'Binding moieties' throughout this invention are molecular complexes that are able to reversibly bind biological molecules or biological compartments. If they are able to bind nucleic acids they are named nucleic acid binding moieties. If they bind double-stranded nucleic acids with a higher affinity than single-stranded nucleic acids they are named double-stranded nucleic acid binding moieties. The phrases double-stranded nucleic acid binding moiety or 'ds-NA binder' are used as equivalents throughout this invention. In the context of this invention the phrase nucleic acid (NA) summarizes deoxyribonucleic acid (DNA) and ribonucleic acid (RNA) as well as nucleic acid analogues like peptide nucleic acids (PNA) or locked nucleic acids (LNA).

Biological compartments are cells or assemblies of cells. Since cells have certain proteins or polymers within their cell membrane, the binding moieties may have an affinity for these molecular structures in order to reversibly bind the cells or assemblies of cells. Additionally, the electrostatic interaction of the negatively charged cell membrane with cationic binding moieties can be utilized to reversibly bind biological compartments.

The binding moieties are coupled to the surface of a solid phase throughout this invention. The surface of this solid phase may be of arbitrary shape and therefore, includes planar surfaces of e.g. a cover slide, the curved surface of e.g. a test tube, a vessel or a pipette tip, the surface of small particles like e.g. magnetic beads or the surface of porous materials like e.g. glass fleeces. Throughout this invention a vessel is a single reaction vessel, like e.g. a Eppendorf cap or a centrifugation tube. As solid phase material all materials are possible within the scope of this invention, as far as the surface of this solid phase material comprises coupling groups for said binding moieties or as far as the surface of this solid phase material may be functionalized with coupling groups for said binding moieties. In some cases it is necessary to provide said surface with a functional coating, before the binding moieties may be coupled. Such a functional coating is e.g. a polymer layer.

In the context of this invention the coupling of the binding moieties to the surface of the solid phase includes covalent bonds like e.g. silane coupling, amide bonds or epoxide coupling, coordinative bindings like e.g. between His-tags and chelators, bioaffine bindings like e.g. a biotin/streptavidin bond.

The 'biological molecules in the sample' are the target molecules potentially present within said sample that should be detected and therefore, they are also named 'target molecules in the sample'. Additionally, certain non-target molecules may be present in said sample that may disturb the detection of the target molecules. These non-target molecules initially present in the sample can not be avoided by the present invention. If the target molecules and the non-target molecules are nucleic acid molecules, they are called target nucleic acid molecules and non-target nucleic acid molecules, respectively. A non-target nucleic acid molecule is a molecule with a different sequence than the target nucleic acid molecule.

Moreover, the reagents necessary to detect said target molecule are solutions that may contain non-target molecules or target molecules or both and both molecules may disturb the detection of the target molecules in the sample, e.g. within a diagnostic test, if they are introduced in said sample as a contamination. Within the scope of this invention, non-target molecules potentially present in the reagents also comprise cells containing target and/or non-target nucleic acid molecules. The non-target molecules and/or the target molecules potentially present in the reagents are summarized by the phrase 'biological molecules potentially present in the reagents' throughout this invention.

Regarding nucleic acids, the target molecules potentially present in the reagents comprise the nucleic acid molecule itself as well as corresponding amplicons and nucleic acids in cells. The phrase 'nucleic acid molecules potentially present in the reagents' is used throughout this invention to summarize all possible combinations of nucleic acid molecules potentially present in the reagents, namely just target nucleic acid molecules, just non-target nucleic acid molecules or both target and non-target nucleic acid molecules.

Reagents include all substances necessary to detect the target molecule in the sample, namely e.g. buffers, enzymes, antibodies, primers, probes, labels, nucleotides and/or oligonucleotides.

The contaminations of said reagents can either be present initially as e.g. non-target molecules and/or target molecules or they may be introduced during one or more of the steps of the detection procedures, e.g. by adding contaminated reagents or simply by contact of the reagents with the surrounding atmosphere. The contaminations of the reagents can introduce non-target molecules as well as target molecules to said sample leading to false positive results.

Within the present invention, possible contaminations of said reagent include cells or assemblies of cells as well and therefore, cells or assemblies of cells are understood as non-target molecules within the scope of the present invention, too. As a prominent example the bacterial contamination of PCR reagents should be mentioned here, since these bacterial contaminations of the reagents can add double-stranded (ds) DNA to the solution as well. If the contaminating bacterium within the reagent is the same as the one that should be verified by detecting a specific target molecule within the sample, this may produce false positive results in a diagnostic test based on a PCR amplification of the target nucleic acid. In this case, target nucleic acid molecules that do not originate from the sample under investigation will contribute to the amplification result of the PCR.

The decontamination of the reagents from non-target molecules and/or target molecules occurs, when the binding moieties are in physical contact with the reagents under certain conditions, whereby the non-target molecules and/or target molecules bind reversibly to the binding moieties.

Throughout this invention, the phrase physical contact shall be understood as any contact between said binding moieties and said reagents under certain conditions of buffer, pressure, temperature, irradiation or mechanical stress. As known to someone skilled in the art, the binding between organic molecules is in general reversible and depending on the conditions of the surrounding buffer solution. Therefore, one has to provide certain conditions in order to guarantee the binding of the non-target molecules and/or target molecules to the binding moieties. Throughout this invention, the physical contact includes immersion of slides or particles in the reagents, filling of e.g. tubes or the flow through of e.g. pipette tips by the reagents.

In the scope of this invention all detection formats are possible for the detection of target molecules as far as they are capable of detecting organic molecules.

In case of DNA, suitable detection methods are known to the expert in the field and are described in standard textbooks as of Sambrook et al.: Molecular Cloning, A Laboratory Manual (2nd Addition, Cold Spring Harbour Laboratory Press, Cold Spring Harbour, NY) or of Ausubel et al.: Current Protocols in Molecular Biology (1987) J. Wiley and Sons, NY).

In case of proteins, suitable assays are described in the text books of Tijssen: Practice and theory of enzyme immunoassays (1985, Elsevier, Amsterdam, Netherlands) and Aslam & Dent: Bioconjugation (2000, Macmillian Reference, London, GB).

For the amplification of target nucleic acid molecules all amplification techniques known to someone skilled in the art are possible within the scope of the present invention, like e.g. PCR, LCR or the replicase amplification.

Another aspect of the invention concerns a solid phase material, whereas double-stranded nucleic acid binding moieties are coupled to the surface of said solid phase material. The complex of the solid phase material and a certain number of binding moieties that are coupled to its surface can be any combination of a solid phase material and binding moieties according to the invention.

Another subject matter of the present invention is the use of said solid phase material for a target nucleic acid amplification in a sample avoiding the amplification of nucleic acid molecules potentially present in the reagents necessary to amplify said target nucleic acid molecules in said sample. The amplification of a target nucleic acid molecule includes any possibility to enlarge the number of a nucleic acid molecule within a sample. Examples are the PCR or LCR amplification providing an exponential growth of the nucleic acid copy number against the cycle number or the replicase amplification providing a linear growth of the nucleic acid copy number against the reaction time.

The invention also concerns a kit comprising the reagents necessary to perform a target nucleic acid amplification and said solid phase material according to the invention.

### Description of the Figures:

- **Figure 1**:: Biotinylated ds-NA binder (MGB)
- **Figure 2**:: Characterization of ds-NA binder (MGBs, peptides & intercalator)
- **Figure 3**:: Decontamination efficiency from bacterial DNA using ds-NA binders coupled to Dynabeads® determined by PCR amplification.
- **Figure 4**:: Decontamination efficiency from hg DNA using ds-NA binders coupled to Dynabeads® determined by PCR amplification.
- **Figure 5**:: Decontamination efficiency from plasmid DNA using three ds-NA binders coupled to Dynabeads® determined by PCR amplification.
- **Figure 6**:: Selectivity evaluation of the Bi-PEG-Distamycin/SA-magnetic bead complex with respect to double-stranded and single-stranded oligonucleotides in a time dependent manner using UV-absorbance.
- **Figure 7**:: Selectivity evaluation of three ds-NA binders with respect to double-stranded and single-stranded oligonucleotides in a time dependent manner using PCR amplification.
- **Figure 8**:: Decontamination of solutions from bacterial DNA using the Bi-PEG-Distamycin/SA-magnetic bead complex and quantification by PCR amplification.
- **Figure 9**:: Decontamination of solutions from bacterial DNA using the Bi-PEG-Distamycin/SA-coated tubes and quantification by PCR amplification.
- **Figure 10**:: Decontamination efficiency for two bacteria concentrations using several bacteria binder/SA-magnetic bead complexes determined by PCR amplification.

### Detailed description of the invention:

One subject matter of the present invention is a method for the detection of biological molecules in a sample that avoids the detection of biological molecules potentially present in the reagents necessary to detect the biological molecules in the sample, comprising the steps of
a) providing a sample potentially comprising said biological molecule,
b) providing binding moieties coupled to the surface of a solid phase,
c) providing reagents necessary to detect the biological molecules,
d) adding said reagents to said sample,
e) detecting the biological molecules in said sample and
wherein in step c) or in step d) or in step c) and d) said reagents are in physical contact with said binding moieties under conditions, whereby said biological molecules potentially present in said reagents bind to said binding moieties coupled to said surface of a solid phase.

Another subject matter of the present invention is a method for the detection of biological molecules in a sample that avoids the detection of biological molecules potentially present in the reagents necessary to detect the biological molecules in the sample, comprising the steps of
a) providing binding moieties coupled to the surface of a solid phase,
b) providing reagents necessary to detect the biological molecules,
c) adding said reagents to the sample potentially comprising said target molecule,
d) detecting the biological molecules in said sample and
wherein in step b) or in step c) or in step b) and c) said reagents are in physical contact with said binding moieties under conditions, whereby said biological molecules potentially present in said reagents bind to said binding moieties coupled to said surface of a solid phase.

Reagents throughout the present invention are all reagents necessary to detect said target molecule and comprise substances like e.g. buffer solutions, enzymes, antibodies, primers, probes, labels, nucleotides and/or oligonucleotides. Since these reagents are added to the sample, it is important to assure that the used reagents are free of contaminations with non-target molecules as well as target molecules in order to avoid false positive results detecting the target molecule within the sample.

The detection methods mentioned above for detecting a target molecule in a sample that avoids the detection of biological molecules potentially present in said reagents, reduce the risk of false positive results due to contaminations.

The biological molecules potentially present in said reagents are selected from the group consisting of target molecules, non-target molecules and target and non-target molecules together. The binding of non-target molecules and/or target molecules to said binding moieties is preferably reversible and therefore, the binding occurs under certain conditions and can be released by changing said conditions. It is preferred that the binding and the release of non-target molecules and/or target molecules can be switched by altering the conditions in terms of buffer concentration, temperature or mechanical stress. Most preferably, the binding of non-target molecules and/or target molecules occurs at room temperature at moderate salt concentrations.

The conditions for binding and the kind of interaction between organic molecules are known to people skilled in the art. In case of proteins, interactions are described in the text books of Tijssen: Practice and theory of enzyme immunoassays (1985, Elsevier, Amsterdam, Netherlands) and Aslam & Dent: Bioconjugation (2000, Macmillian Reference, London, GB). The interaction of DNA with other organic molecules is described in standard textbooks as of Sambrook et al.: Molecular Cloning, A Laboratory Manual (2nd Addition, Cold Spring Harbour Laboratory Press, Cold Spring Harbour, NY) or of Ausubel et al.: Current Protocols in Molecular Biology (1987, J. Wiley and Sons, NY).

Binding of ds DNA to ds-NA binders like minor groove binders or intercalators can be performed in salt containing solutions like sodium chloride, potassium chloride, magnesium chloride or Tris buffer, whereas the binding affinity is higher at lower salt concentrations, preferably a salt concentration of 10 - 100 mM is used. Moreover, the binding step is preferably performed at room temperature at non-denaturing pH, preferably at pH 6 - 8 (Zimmer, G. et al., Prog. Biophys. Molec. Biol. 47 (1986) 37-112; Pauluhn, J. et al., Ber. Bunsenges. Phys. Chem. 82 (1978) 1265-1278).

For the binding of ds DNA to ds-DNA binding antibodies TBS buffer can be used, preferably 25 mM Tris and 150 mM sodium chloride at pH 7,4 and room temperature (Di Pietro, S.M. et al., Biochemistry 42 (2003) 6218-6227). The affinity of the protein-DNA binding decreases with increasing salt concentration (Record, M.T. et al., J. Mol. Biol. 107 (1976) 145-158).

Additionally, conditions suitable to bind double-stranded DNA to the different ds-DNA binder can be found in the articles as well as in the patents cited in the chapter "Prior Art Background" and in the examples of this invention.

In a preferred method according to the invention said conditions to bind said biological molecules potentially present in said reagents to said binding moieties coupled to said surface of a solid phase comprise a salt concentration of 10 - 200 mM, most preferably of 10 - 50 mM, a non-denaturing pH, most preferably pH 6 - 8, an incubation time of 5 - 90 minutes, most preferably of 60 minutes, and room temperature.

In another preferred method according to the invention said reagents necessary to detect said biological molecules in the sample are mixed prior to the adding step, whereby said reagents are in physical contact with said binding moieties under conditions, whereby said biological molecules potentially present in said reagents bind to said binding moieties coupled to said surface of a solid phase.

Since in general two or more reagents are necessary in a certain ratio to perform the detection of the target molecule, it may be advantageous to mix said reagents prior to the addition to the sample, whereby the reagents are in physical contact with said binding moieties, preferably at all times. In another embodiment of the invention, the reagents are added successively to the sample in appropriate amounts, whereby each reagent is in physical contact with said binding moieties, preferably at all times.

In a preferred method of the invention said surface of a solid phase is the inner surface of a vessel or a pipette tip.
In this embodiment of the invention, the reagents necessary to detect said target molecules are provided, maintained and/or mixed in a vessel with binding moieties coupled to its surface prior to the addition to the sample. If the reagents are transferred from one vessel to another or if the reagents are added finally to the sample, in one embodiment of the invention a pipette tip with binding moieties coupled to its surface is used. The solid phases functionalized with the binding moieties according to the present invention are preferably plastic devices, like e.g. tubes or pipette tips, or glass devices like e.g. reaction vessels.

In another preferred method of the invention said surface of a solid phase is the surface of beads or the surface of a porous material.

In this embodiment of the invention, beads with binding moieties coupled to the surfaces of said beads are added to a vessel containing a certain reagent. Providing the appropriate conditions for a certain incubation time, the non-target molecules and/or the target molecules bind to the binding moieties and the supernatant comprises the reagent that is preferably free of non-target molecules and of target molecules. Most preferably, these beads are magnetic beads, like the commercial magnetic Dynabeads® (Dynal Biotech S.A., Oslo, Norway).

In another embodiment of the invention, a porous material with binding moieties coupled to the surfaces of said porous material is used to get rid of non-target molecules and/or target molecules within the reagents. Preferably, the porous material is formed as a filter, whereas said filter can be combined with e.g. a syringe in order to remove non-target molecules and/or target molecules from the reagents during the transfer form one vessel to another or to the sample. The porous solid phases possible to couple the binding moieties according to the present invention are preferably porous inorganic materials, like glass fleeces or controlled-pore-glass. An alternative are porous plastics like polyethylene (PE) or polypropylene (PP) or polyethylenterephthalate (PET), polyacrylnitrile (PAT), polyvinylidendifluoride (PVDF) or polystyrene. In another alternative embodiment of the invention, porous organic materials are used, like e.g. porous polymer or copolymer material.

In a preferred embodiment of the method, the binding moieties according to the invention are coupled to said surface of a solid phase via a covalent bond or via a bioaffine bond, preferably a biotin/streptavidin bond.

The coupling of said binding moieties to the surface of said solid phase includes covalent bonds like e.g. silane coupling to hydroxy surfaces or amino coupling to epoxide, thiol coupling to metals like gold, coordinative bindings like e.g. between His-tags and chelators, bioaffine bindings like e.g. a biotin/streptavidin or biotin/avidin bond. In another embodiment of the invention, the surface of said solid phase is covered by a polymer layer comprising the coupling sites for the binding moieties.

In a preferred embodiment of the invention, the binding affinity of said binding moieties realize a reduction of the biological molecule content in the reagents by at least a factor of 10², preferably by at least a factor of 10³.

The biological molecule content in the reagents comprises the content of target molecules and/or the content of non-target molecules in the reagents, depending on the type of nucleic acid molecules present in said reagents.

The phrase 'binding affinity' is used throughout the invention as a qualitative measure for the ability to bind non-target molecules and/or target molecules. The ability of a certain binding moiety to reduce the content of non-target molecules and/or target molecules may be dependent on the conditions of the environment, namely e.g. the buffer composition or the temperature. Within the scope of this invention, the combination of binding moiety and environment conditions are adjusted to realize a reduction of the non-target molecule content and/or of the target molecule content within the reagents by at least a factor of 10², more preferably by at least a factor of 10³.

In an also preferred embodiment, the method according to the invention further comprises the following steps
f) eluting the biological molecules associated with said binding moieties and
g) detecting said eluted biological molecules.

Since the binding of non-target molecules and/or of target molecules to the binding moieties according to the invention is reversible, it is possible to detect the bound molecules after the decontamination in order to learn about the contaminations within the used reagents or of the contaminations introduced during the preparation steps. Said elution occurs, if the binding condition in terms of buffer concentration, temperature, pH, denaturing reagents or mechanical stress are changed. It is preferred that the release of non-target molecules and/or of target molecules from said binding moieties is performed at moderate temperatures and salt concentrations at alkaline pH. In case of an electrostatic interaction between the binding moieties and the non-target molecules and/or target molecules, the release can be obtained by increasing the salt concentration and the corresponding enhancement of screening effects. In general, the coupling of non-target molecules and/or of target molecules to the binding moieties can be raptured by increasing the temperature.

In yet another preferred method according to the invention said target molecules and said non-target molecules are nucleic acid molecules, most preferably said target molecules and said non-target molecules in the reagents are double-stranded nucleic acid molecules.

In a preferred embodiment of the invention, the binding moieties are moieties capable of binding double-stranded nucleic acid molecules.
If the binding moieties bind double-stranded nucleic acid molecules they are named double-stranded nucleic acid binding moieties. The phrases double-stranded nucleic acid binding moiety or 'ds-NA binder' are used as equivalents throughout this invention.

In a more preferred embodiment of the invention, the binding moieties are polycationic entities, minor groove binders, intercalators or anti double-stranded nucleic acid antibodies.
A polycationic entity is a large molecule having multiple charges. In case of a polymer or a peptide this polycationic entity comprises a certain amount of monomers, each monomer having a positive charge or a negative charge or is neutral. Therefore, such a polycationic entity is characterized by its net charge, which is the sum of all monomer charges. Examples are peptides or polyamide derivatives. Minor groove binders (MGBs), like e.g. Distamycin or methyl-imidazol-polyamide derivatives, are molecules that bind to the minor groove of a double-stranded nucleic acid. Intercalators, like e.g. Actinomycin D or ethidium bromide, are molecules that bind between the stacked base pairs of a double-stranded nucleic acid. Anti-ds DNA antibodies are known to selectively bind only to double-standed DNA and not to single-stranded DNA. These kind of ds-NA binders are known to someone skilled in the art and additional informations are included in the chapter 'prior art background'.

In another embodiment according to the invention, said detection of the target molecule in the sample is based on a nucleic acid amplification reaction.
Suitable DNA detection methods are known to the expert in the field and are described in standard textbooks as of Sambrook et al.: Molecular Cloning, A Laboratory Manual (2nd Addition, Cold Spring Harbour Laboratory Press, Cold Spring Harbour, NY) or of Ausubel et al.: Current Protocols in Molecular Biology (1987) J. Wiley and Sons, NY). The detection methods may include but are not limited to the binding or intercalating of specific dyes as ethidium bromide which intercalates into the double-stranded DNA and changes its fluorescence thereafter. The purified DNA may also be separated by electrophoretic methods optionally after a restriction digest and visualized thereafter. There are also probe-based assays which exploit the oligonucleotide hybridisation to specific sequences and subsequent detection of the hybrid. It is also possible to sequence the DNA after further steps known to the expert in the field. Other methods apply a diversity of DNA sequences to a silicon chip to which specific probes are bound and yield a signal when a complementary sequence binds.

The amplification of a target nucleic acid molecule includes any possibility to enlarge the copy number of a nucleic acid molecule within a sample. Examples are the PCR or LCR amplification providing an exponential growth of the nucleic acid copy number against the cycle number or the replicase amplification providing a linear growth of the nucleic acid copy number against the reaction time. Other possible amplification reactions are already described in the chapter 'Prior Art Background'.

All preparation steps prior to said nucleic acid amplification, like e.g. preparing of buffer solutions, diluting of stock solutions, providing of enzymes, nucleotides, primers and probes, mixing of ingredients and/or pipetting the reagents into the reaction vessel for amplification, comprise a certain risk of contaminating the sample. Therefore, as many of these preparation steps prior to said nucleic acid amplification as possible should be performed with the aid of the binding moieties coupled to the surface of a solid phase.

Particularly preferred detection methods for DNA can be done in so-called "homogeneous" assays. A "homogeneous" assay system comprises reporter molecules or labels which generate a signal while the target sequence is amplified. An example for a "homogeneous" assay system is the TaqMan® system that has been detailed in US 5,210,015, US 5,804,375 and US 5,487,972. Briefly, the method is based on a double-labelled probe and the 5'-3' exonuclease activity of Taq DNA polymerase. The probe is complementary to the target sequence to be amplified by the PCR process and is located between the two PCR primers during each polymerisation cycle step. The probe has two fluorescent labels attached to it. One is a reporter dye, such as 6-carboxyfluorescein (FAM), which has its emission spectra quenched by energy transfer due to the spatial proximity of a second fluorescent dye, 6-carboxy-tetramethyl-rhodamine (TAMRA). In the course of each amplification cycle, the Taq DNA polymerase in the process of elongating a primed DNA strand displaces and degrades the annealed probe, the latter due to the intrinsic 5'-3' exonuclease activity of the polymerase. The mechanism also frees the reporter dye from the quenching activity of TAMRA. As a consequence, the fluorescent activity increases with an increase in cleavage of the probe, which is proportional to the amount of PCR product formed. Accordingly, the amplified target sequence is measured by detecting the intensity of released fluorescence label.

A similar principle of energy transfer between fluorescent dye molecules applies to "homogeneous" assays using so-called "molecular beacons" (US 6,103,476). These are hairpin-shaped nucleic acid molecules with an internally quenched fluorophore whose fluorescence is restored when they bind to a target nucleic acid (US 6,103,476). They are designed in such a way that the loop portion of the molecule is a probe sequence complementary to a region within the target sequence of the PCR process. The stem is formed by the annealing of complementary arm sequences on the ends of the probe sequence. A fluorescent moiety is attached to the end of one arm and a quenching moiety is attached to the end of the other arm. The stem keeps these two moieties in close proximity to each other, causing the fluorescence of the fluorophore to be quenched by energy transfer. Since the quencher moiety is a non-fluorescent chromophore and emits the energy that it receives from the fluorophore as heat, the probe is unable to fluoresce. When the probe encounters a target molecule, it forms a hybrid that is longer and more stable than the stem hybrid and its rigidity and length preclude the simultaneous existence of the stem hybrid. Thus, the molecular beacon undergoes a spontaneous conformational reorganisation that forces the stem apart, and causes the fluorophore and the quencher to move away from each other, leading to the restoration of fluorescence which can be detected.

More examples for "homogeneous" assay systems are provided by the formats used in the LightCycler® instrument (see e.g. US 6,174,670), some of them are called "kissing probe" formats sometimes. Again, the principle is based on two interacting dyes which, however, are characterised in that the donor-dye excites an acceptor-dye by fluorescence resonance energy transfer. An exemplified method uses two modified oligonucleotides as hybridisation probes, which hybridise to adjacent internal sequences of the target sequence of the PCR process. The 5'-located modified oligonucleotide has a donor-dye as a label at its 3' end. The 3'-located modified oligonucleotide has an acceptor-dye at its 5' end. Following the head-to-tail-oriented annealing of the two modified oligonucleotides to the target sequence in the course of an amplification cycle, donor and acceptor dye are brought in close proximity. Upon specific excitation of the donor dye by means of a monochromatic light pulse, acceptor dye fluorescence is detected providing a measure for the amount of PCR product formed.

Another assay format is the so-called "array" format. An "array" is an arrangement of addressable locations on a device (e.g. US 5,143,854, US 6,022,963, US 6,156,501, WO 90/15070, WO 92/10092). The number of locations can range from several to at least hundreds of thousands. Most importantly, each location represents a totally independent reaction site. Each location carries a nucleic acid as e.g. an "oligomeric compound", which can serve as a binding partner for a second nucleic acid, in particular a target nucleic acid. Methods for the manufacturing thereof are described in EP-A-0 476 014, Hoheisel, J. D., TIBTECH 15 (1997) 465-469, WO 89/10977, WO 89/11548, US 5,202,231, US 5,002,867, WO 93/17126. Further developments have provided methods for making very large arrays of oligonucleotide probes in very small areas (US 5,143,854, WO 90/15070, WO 92/10092). Microfabricated arrays of large numbers of oligonucleotide probes, called "DNA chips" offer great promise for a wide variety of applications (e.g. US 6,156,501 and US 6,022,963). The basic steps of the method are that nucleic acid from control and treatment samples is isolated and labeled with different fluorescent dyes incorporated during an amplification process. In more detail, this is performed according to the method described in US 5,545,522; US 5,716,785; US 5,891,636 and US 6,291,170, whereby double stranded cDNA is synthesized with a primer comprising the bacterial T7-Promoter and labeled RNA is transcribed in the presence of ribonucleoside triphosphates, whereby labels are attached to some of the nucleoside triphosphates. These labeled nucleic acids are then optionally fragmented, mixed and hybridized to the arrayed oligomeric compounds.

An optical device is then used to measure the relative intensities of each dye for each individual spot. The ratio of fluorescence levels between the two probes indicates the relative gene expression between the samples. By these processes researchers can evaluate an entire set of genes simultaneously rather than looking at the effects of single genes one at a time. High differential expression of specific genes can then be followed up by conventional means such as northern blot or quantitative real-time PCR. Data from multiple experiments can be combined in order to assign functional information to genes of otherwise unknown function. Genes showing similar expression profiles across differing states are likely to participate in common physiological or metabolic pathways. Cluster analysis programs have been developed which allow detection of co-expressed groups of genes reflecting information on function. In yet another embodiment according to the invention, said binding moieties are moieties capable of binding biological compartments.

In another embodiment of the invention, the contaminations within the reagents are biological compartments and the binding moieties are able to bind these biological compartments. Biological compartments are cells or assemblies of cells. Cells or assemblies of cells that are potentially present in the reagents and contain target and/or non-target nucleic acid molecules are non-target molecules within the scope of this invention. Since cells have certain proteins or polymers within their cell membrane, the binding moieties of this embodiment of the invention have a certain affinity for these molecular structures in order to reversibly bind the cells or assemblies of cells. Possible binding moieties for this embodiment of the invention comprises antibodies for membrane compounds or cell binding proteins, preferably Fibronectin or functional parts of those binding proteins.

In another embodiment, the binding moieties for binding cells or assemblies of cells are binding moieties that assemble with the membrane structure, preferably dye molecules or amphiphilic molecules. Additionally, minor groove binder (MGB) are applicable as binding moieties for binding cells or assemblies of cells. Examples for dyes comprise Crystal violet, methylene blue and Safranin O that are functionalized in order to couple to solid surfaces. Examples for amphiphilic molecules capable of binding to cell membranes comprise e.g sodium dodecyl sulfate (SDS), cholesterol or lauroyl-lysin, also functionalized in order to couple to solid surfaces. Suitable MGBs are e.g. Distamycin or methyl-imidazol-polyamide derivatives.

In yet another embodiment, the binding moieties for binding cells or assemblies of cells are polycationic entities. These polycationic entities, preferably polycationic peptides or polymers, bind cells by electrostatic interactions with the multiple negative charges of the cell membranes. These polycationic entities are functionalized in order to couple to solid surfaces, too.

In another preferred embodiment of the invention, said binding moieties are moieties capable of binding double-stranded nucleic acid molecules and biological compartments. A possible variant of this embodiment of the invention is the combination of binding moieties that are specific for cells or assemblies of cells with binding moieties that are specific for double-stranded nucleic acid molecules. Therefore, this embodiment of the invention can be realized with two or more different binding moieties or with one type of binding moiety that is able to bind both double-stranded nucleic acid molecules and biological compartments. The method according to this embodiment of the invention is able to bind contaminations based on cells or assemblies of cells and on double-stranded nucleic acid molecules potentially present in said reagents in a single preparation step.

Another aspect of the invention concerns a method to amplify a target nucleic acid molecule, comprising the steps of
a) providing a sample potentially comprising target nucleic acid molecules,
b) providing nucleic acid binding moieties coupled to the surface of a solid phase,
c) providing reagents necessary to amplify said target nucleic acid molecules,
d) adding said reagents to said sample,
e) amplifying said target nucleic acid molecules in said sample and
wherein in step c) or in step d) or in step c) and d) said reagents are in physical contact with said nucleic acid binding moieties under conditions, whereby said nucleic acid molecules potentially present in said reagents bind to said nucleic acid binding moieties coupled to said surface of a solid phase.

Another aspect of the invention concerns a method to amplify a target nucleic acid molecule avoiding the amplification of nucleic acid molecules potentially present in said reagents necessary to amplify said target nucleic acid molecules in the sample, comprising the steps of
a) providing a sample potentially comprising target nucleic acid molecules,
b) providing nucleic acid binding moieties coupled to the surface of a solid phase,
c) providing reagents necessary to amplify said target nucleic acid molecules,
d) adding said reagents to said sample,
e) amplifying said target nucleic acid molecules in said sample and
wherein in step c) or in step d) or in step c) and d) said reagents are in physical contact with said nucleic acid binding moieties under conditions, whereby said nucleic acid molecules potentially present in said reagents bind to said nucleic acid binding moieties coupled to said surface of a solid phase.

Yet another aspect of the invention concerns a method to amplify a target nucleic acid molecule in a sample comprising the steps of
a) providing nucleic acid binding moieties coupled to the surface of a solid phase,
b) providing reagents necessary to amplify said target nucleic acid molecules,
c) adding said reagents to the sample potentially comprising target nucleic acid molecules,
d) amplifying said target nucleic acid molecules in said sample and
wherein in step b) or in step c) or in step b) and c) said reagents are in physical contact with said nucleic acid binding moieties under conditions, whereby said nucleic acid molecules potentially present in said reagents bind to said nucleic acid binding moieties coupled to said surface of a solid phase.

Yet another aspect of the invention concerns a method to amplify a target nucleic acid molecule in a sample avoiding the amplification of nucleic acid molecules potentially present in said reagents necessary to amplify said target nucleic acid molecules in the sample, comprising the steps of
a) providing nucleic acid binding moieties coupled to the surface of a solid phase,
b) providing reagents necessary to amplify said target nucleic acid molecules,
c) adding said reagents to the sample potentially comprising target nucleic acid molecules,
d) amplifying said target nucleic acid molecules in said sample and
   wherein in step b) or in step c) or in step b) and c) said reagents are in physical contact with said nucleic acid binding moieties under conditions, whereby said nucleic acid molecules potentially present in said reagents bind to said nucleic acid binding moieties coupled to said surface of a solid phase.

To amplify a nucleic acid molecule several reagents are necessary, comprising buffer solutions, enzyms, primers, probes, labels and/or nucleotides. Since these reagents are added to the sample, it is important to assure that the used reagents are free of contaminations with non-target nucleic acid molecules as well as nucleic acid target molecules in order to avoid false positive results detecting the target molecule within the sample.

The nucleic acid molecules potentially present in said reagents are selected from the group consisting of only target nucleic acid molecules, only non-target nucleic acid molecules and target and non-target nucleic acid molecules together. The amplification methods mentioned above for a target nucleic acid amplification in a sample that avoids the amplification of non-target nucleic acid molecules and/or target nucleic acid molecules potentially present in said reagents, reduce the risk of false positive results due to contaminations.

It is preferable that the binding of non-target nucleic acid molecules and/or target nucleic acid molecules occurs at conditions comprising room temperature and moderate salt concentrations as described before.

In another preferred method to amplify a target nucleic acid molecule according to the invention, said reagents necessary to amplify said target nucleic acid molecules are mixed prior to the adding step d), whereby said reagents are in physical contact with said nucleic acid binding moieties under conditions, whereby nucleic acid molecules potentially present in said reagents bind to said nucleic acid binding moieties coupled to said surface of a solid phase.

As described before regarding the detection of target molecules, more than one reagent is necessary to amplify the target nucleic acid and therefore, it may be advantageous to mix said reagents prior to the addition to the sample, whereby the reagents are in physical contact with said binding moieties, preferably at all times. The detection of the amplified target nucleic acid can be performed during the amplification, e.g. in real-time PCR, or after the amplification.

In a preferred method to amplify a target nucleic acid molecule according to the invention, said surface of a solid phase is the inner surface of a vessel or a pipette tip.

In another preferred method to amplify a target nucleic acid molecule according to the invention, said surface of said solid phase is the surface of beads or the surface of a porous material.

As described before regarding the detection of target molecules the reagents necessary to amplify said target nucleic acid molecules are preferably provided, maintained and/or mixed in a vessel with binding moieties coupled to its surface prior to the addition to the sample. If the reagents are transferred from one vessel to another or if the reagents are added finally to the sample, in one embodiment of the invention a pipette tip with binding moieties coupled to its surface is used. The beads with binding moieties coupled to the surfaces of said beads can be added e.g. to a vessel containing a certain reagent to bind non-target nucleic acid molecules and/or target nucleic acid molecules and to obtain a supernatant that is preferably free of contaminations. The porous material with binding moieties coupled to the surfaces of said porous material can be used to get rid of contaminations within the reagents, too. Preferably, the porous material is formed as a filter, wheras said filter can be combined with a syringe in order to remove non-target nucleic acid molecules and/or target nucleic acid molecules from the reagents during the transfer form one vessel to another or finally to the sample.

In a preferred method to amplify a target nucleic acid molecule according to the invention, said non-target nucleic acid molecules and said target nucleic acid molecules in the reagents are double-stranded nucleic acid molecules, preferably double-stranded DNA molecules.

In another preferred method to amplify a target nucleic acid molecule according to the invention, said nucleic acid binding moieties are double-stranded nucleic acid binding moieties.

Possible double-stranded nucleic acid binding moieties are already described before regarding the detection of target molecules. In order to enable the binding of the double-stranded nucleic acid molecules to the ds-NA binders, the temperature has to be kept below the melting temperature of the hybrids.

In a more preferred method to amplify a target nucleic acid molecule according to the invention, said binding affinity of said double-stranded nucleic acid binding moieties to single-stranded nucleic acid molecules is lower than to double-stranded nucleic acid molecules.

As mentioned before, the binding affinity is a qualitative measure for the ability of a binding moiety to bind non-target molecules and/or target molecules throughout this invention. Note, that the binding affinity may be dependent on the conditions of the environment, namely e.g. the buffer composition or the temperature. In case of the ds-NA binder that should distinguish between double-stranded nucleic acid and single-stranded nucleic acids, the binding affinity towards these two molecule types must be different. Therefore, within the scope of this invention, the combination of ds-NA binder and the conditions of the environment is adjusted to realize such an affinity difference.

A method to determine this affinity difference of a ds-DNA binder in a quantitative way is to provide two samples comprising the same amount of single-stranded and double-stranded DNA molecules, respectively. After a certain incubation time of both samples with the same number of ds-DNA binder under equal reaction conditions, the remaining amount of both the single-stranded and the double-stranded DNA molecules in the samples is quantified. The ratio of the remaining amounts in the samples is the affinity difference of a ds-DNA binder to single-stranded and double-stranded DNA.

More general, the affinity difference of the ds-DNA binder must be large enough to provide the possibility to bind a small number of long, double-stranded nucleic acid molecules present in a reagent containing a large number of short, single-stranded primer molecules in an efficient manner. If the affinity difference of the ds-DNA binder is not sufficient, the short single-stranded primer molecules will occupy all binding moieties and the long, double-stranded nucleic acid molecules will remain in the reagent (see Example 8).

In another preferred method to amplify a target nucleic acid molecule according to the invention, said binding affinity of said double-stranded nucleic acid binding moieties realize a reduction of the double-stranded nucleic acid content within the reagents by at least a factor of 10², more preferably by at least a factor of 10³.

The double-stranded nucleic acid content comprises the content of double-stranded target nucleic acid molecules and/or the content of double-stranded non-target nucleic acid molecules in the reagents, depending on the type of nucleic acid molecules present in said reagents.

According to another embodiment of the method to amplify a target nucleic acid molecule, said double-stranded nucleic acid binding moieties are polycationic entities, minor groove binders, intercalators or anti double-stranded nucleic acid antibodies.

According to a preferred embodiment of the method to amplify a target nucleic acid molecule, said polycationic entities are nucleic acid binding polyamide derivatives.

According to a more preferred embodiment of the method to amplify a target nucleic acid molecule, said nucleic acid binding polyamide derivatives have a positive net charge, preferably at least with 0.1 positive net charges per monomer, more preferably at least with 0.2 positive net charges per monomer.

As defined before, a polycationic entity is a large molecule having multiple charges. In case of a polymer or a peptide this polycationic entity comprises a certain amount of monomers, each monomer having a positive charge, a negative charge or is neutral. Therefore, such a polycationic entity is characterized by its net charge, which is the sum of all monomer charges. In order to compare polycationic entities with a different amount of monomeric units, the net charge is normalized on this number of monomeric units, providing the quantity 'positive net charges per monomer'. These polycationic entity provide a certain selectivity between double-stranded and single-stranded nucleic acids, namely the polycationic entities have a higher affinity towards double-stranded nucleic acids compared to single-stranded nucleic acids. Without being bound to theory, this effect may originate from the difference in molecular charge of double-stranded and single-stranded nucleic acids. In a preferred embodiment of the invention, where long double-stranded nucleic acids (e.g. bacterial DNA) are present in a reagent containing short single-stranded nucleic acids (e.g. primers), the selectivity based on charge differences between the double-stranded and the single-stranded nucleic acids in the reagents is even more pronounced.

According to a preferred embodiment of the method to amplify a target nucleic acid molecule, said minor groove binders are Distamycin, Netropsin, methyl-imidazol-polyamide derivatives or 4',6-diamidino-2-phenylindole (DAPI) derivatives.

According to another preferred embodiment of the method to amplify a target nucleic acid molecule, said intercalators are acridine derivatives, preferably acriflavine derivatives or phenanthridinium compounds, preferably ethidium bromide derivatives.

The minor groove binders and intercalators mentioned above as well as other molecules that can be used for the present invention, too are e.g. described in the text book of Demeunynck et al (Eds.): DNA and RNA binders (Vol. 1 & 2, 2003, Wiley-VCH, Weinheim).

In another preferred method to amplify a target nucleic acid molecule according to the invention, said double-stranded nucleic acid binding moieties are coupled to said surface of the solid phase via a covalent bond or via a bioaffine bond, preferably a biotin/streptavidin bond.

In another preferred method to amplify a target nucleic acid molecule according to the invention, biotinylated double-stranded nucleic acid binding moieties are bound to the streptavidin coated surface of the solid phase.

Using this standard system of a bioaffine bond has the advantage that several solid phase materials already functionalized with Streptavidin are commercially available. For example Streptavidin coated Dynabeads® M-280 (Dynal Biotech S.A., Prod. No. 112.06, Oslo, Norway) or Streptavidin-coated PCR tubes (Roche Cat. No. 1741772, Roche Diagnostics GmbH, Mannheim). In addition, the modification of different organic molecules with a biotin group is widely used in the art (see text book of Kessler (Ed.): Nonradioactive labeling and detection of biomolecules, 1992, Springer Verlag, Heidelberg).

In another preferred method to amplify a target nucleic acid molecule according to the invention, the method further comprises the steps
f) eluting the nucleic acid molecules associated with said nucleic acid binding moieties and
g) detecting said eluted nucleic acid molecules.

This preferred method according to the invention provides the opportunity to detect the bound non-target nucleic acid molecules and/or target nucleic acid molecules after the decontamination in order to learn about the contamination within the reagents used or of the contamination introduced during the detection procedures. The detection of the eluted nucleic acid molecules can be performed with techniques known to someone skilled in the art, e.g. PCR amplification or certain array technologies.

In a more preferred method to amplify a target nucleic acid molecule according to the invention, the elution in step f) comprises temperature treatment, change of ionic strength, pH modification or denaturing reagents.

Said elution of bound non-target nucleic acid molecules and/or target nucleic acid molecules can be performed by temperature treatment, change of ionic strength, pH modification and/or denaturing reagents. In case of a ds-NA binder as binding moiety and consequently double-stranded DNA as bound molecules, the elution is equivalent to the denaturing of the DNA hybrid, whereas increasing the temperature above the melting temperature or alkaline pH conditions are preferred.

In yet another preferred method to amplify a target nucleic acid molecule according to the invention, the amplification of said target nucleic acid is a PCR amplification.

In an also preferred method to amplify a target nucleic acid molecule according to the invention, said reagents comprise oligonucleotides, nucleotides, enzymes and buffer solutions.

In more preferred method to amplify a target nucleic acid molecule according to the invention, said oligonucleotides comprise primers and probes and said enzymes comprise DNA-polymerases and uracil-N-glycosylase (UNG).

In another embodiment of the method to amplify a target nucleic acid molecule according to the invention, the amplification of said target nucleic acid molecule is a nucleic acid amplification in a test for microbiological infection of a sample.

Examples for a microbiological infection test of a sample are sepsis tests for detecting Gram-positive bacteria, like e.g. staphylococcus aureus or streptococcus pneumoniae, and Gram-negative bacteria, like e.g E.choli or Enterobacter aerogenes. Such a test is known to someone skilled in the art and comprises several steps starting from obtaining the biological samples potentially containing the bacterial cells, lysis of the cells and finally isolating the genetic material for amplification.

An other aspect of the invention concerns a solid phase material, whereas double-stranded nucleic acid binding moieties are coupled to the surface of said solid phase material.

The complex out of the solid phase material and a certain number of ds-NA binders that are coupled to its surface can be any combination of a solid phase material and binding moieties according to the invention. The surface of this solid phase may be of arbitrary shape and therefore, includes planar surfaces of e.g. a cover slide, the curved surface of e.g. a test tube, a vessel or a pipette tip, the surface of small particles like e.g. magnetic beads or the surface of porous materials like e.g. glass fleeces. Throughout this invention a vessel is a single reaction vessel, like e.g. a Eppendorf cap or a centrifugation tube. As solid phase material all materials are possible within the scope of this invention, as far as the surface of this solid phase material comprises coupling groups for said binding moieties or as far as the surface of this solid phase material may be functionalized with coupling groups for said binding moieties. In some cases it is necessary to provide said surface with a functional coating, before the binding moieties may be coupled. Such a functional coating is e.g. a polymer layer.

Yet another aspect of the invention concerns a solid phase material, whereas double-stranded nucleic acid binding moieties are coupled to the surface of said solid phase material and whereas said solid phase material is a vessel, a bead or a pipette tip.

According to another preferred embodiment of the invention, said solid phase material is a bead or a porous material.

In a preferred embodiment of the solid phase material according to the invention, the binding affinity of said double-stranded nucleic acid binding moieties to single-stranded nucleic acid molecules is lower compared to double-stranded nucleic acid molecules.

In another preferred embodiment of the solid phase material according to the invention, the binding affinity of said double-stranded nucleic acid binding moieties realize a reduction of the double-stranded nucleic acid content within the reagents by at least a factor of 10², preferably at least a factor of 10³.

According to another preferred embodiment of the invention, said double-stranded nucleic acid binding moieties are polycationic entities, minor groove binders, intercalators or anti double-stranded nucleic acid antibodies.

According to a more preferred embodiment of the solid phase material according to the invention, said polycationic entities are nucleic acid binding polyamide derivatives.

According to a even more preferred embodiment of the solid phase material according to the invention, said nucleic acid binding polyamide derivatives have a positive net charge, preferably at least with 0.1 positive net charges per monomer, more preferably at least with 0.2 positive net charges per monomer.

According to yet another preferred embodiment of the invention, said minor groove binders are Distamycin, Netropsin, methyl-imidazol-polyamide derivatives or 4',6-diamidino-2-phenylindole (DAPI) derivatives.

According to yet another preferred embodiment of the invention, said intercalators are acridine derivatives, preferably acriflavine derivatives or phenanthridinium compounds, preferably ethidium bromide derivatives.

In another preferred embodiment of the solid phase material according to the invention, said double-stranded nucleic acid binding moieties are coupled to said surface of a solid phase via a covalent bond or via a bioaffine bond, preferably a biotin/streptavidin bond.

In a more preferred embodiment of the solid phase material according to the invention, biotinylated double-stranded nucleic acid binding moieties are bound to the streptavidin coated surface of the solid phase.

An other aspect of the invention is a solid phase material, whereas polycationic entities or intercalators are coupled to the surface of said solid phase material as double-stranded nucleic acid binding moieties.

In a preferred embodiment of the solid phase material according to the invention, biotinylated polycationic entities or biotinylated intercalators are bound to the streptavidin coated surface of the solid phase as double-stranded nucleic acid binding moieties.

Yet another aspect of the invention is a solid phase material, whereas binding moieties are coupled to the surface of said solid phase material that are able to bind biological compartments and whereas said solid phase material is a vessel or a pipette tip.

As mentioned before, biological compartments are cells or assemblies of cells. Since cells have certain proteins or polymers within their cell membrane, the binding moieties of this embodiment of the invention have a certain affinity for these molecular structures in order to reversibly bind the cells or assemblies of cells.

In a preferred embodiment of the solid phase material to bind biological compartments according to the invention, said binding moieties are minor groove binders, antibodies, dyes, amphiphilic entities or polycationic entities.

Possible binding moieties for this embodiment of the invention comprises antibodies for membrane compounds or cell binding proteins, preferably Fibronectin or functional parts of those binding proteins. Also possible are binding moieties that assemble with the membrane structure, preferably dye molecules or amphiphilic molecules. Additionally, minor groove binder (MGB) are applicable as binding moieties for binding cells or assemblies of cells. Examples for dyes comprise Crystal violet, methylene blue and Safranin O that are functionalized in order to couple to solid surfaces. Examples for amphiphilic molecules capable of binding to cell membranes comprise e.g sodium dodecyl sulfate (SDS), cholesterol or lauroyl-lysin, also functionalized in order to couple to solid surfaces. Suitable MGBs are e.g. Distamycin or methyl-imidazol-polyamide derivatives. Moreover, polycationic entities can be used as binding moieties for binding cells or assemblies of cells. These polycationic entities, preferably polycationic peptides or polymers, bind cells by electrostatic interactions with the multiple negative charges of the cell membranes. These polycationic entities are functionalized in order to couple to solid surfaces, too.

In another preferred embodiment of the solid phase material to bind biological compartments according to the invention, biotinylated binding moieties are bound to the streptavidin coated surface of the solid phase.

A further aspect of the invention is a solid phase material, whereas dyes, amphiphilic entities, polycationic entities or minor groove binders are coupled to the surface of said solid phase material as binding moieties for biological compartments. It is preferred that said binding moieties are biotinylated and that said biotinylated binding moieties are bound to the streptavidin coated surface of the solid phase.

Another aspect of the invention concerns a solid phase material, whereas binding moieties are coupled to the surface of said solid phase material that are able to bind double-stranded nucleic acid molecules and biological compartments.

In this embodiment of the invention, said binding moieties are moieties capable of binding double-stranded nucleic acid molecules and biological compartments. A possible variant of this embodiment of the invention is the combination of binding moieties that are specific for cells or assemblies of cells with binding moieties that are specific for double-stranded nucleic acid molecules. Therefore, this embodiment of the invention can be realized with two or more different binding moieties or with one type of binding moiety that is able to bind both double-stranded nucleic acid molecules and biological compartments. The solid phase material according to this embodiment of the invention is able to bind contaminations based on cells or assemblies of cells and on double-stranded nucleic acid molecules potentially present in said reagents in a single preparation step.

In a preferred embodiment of the solid phase material to bind double-stranded nucleic acid molecules and biological compartments, said solid phase material is a bead, a porous material, a vessel or a pipette tip.

In another preferred embodiment of the solid phase material to bind double-stranded nucleic acid molecules and biological compartments, two or more kinds of binding moieties are coupled to the surface of said solid phase material.

In a more preferred embodiment of the solid phase material to bind double-stranded nucleic acid molecules and biological compartments, said two or more binding moieties are choosen from the group consisting of minor groove binders, intercalators, antibodies, dyes, amphiphilic entities or polycationic entities.

In yet another preferred embodiment of the solid phase material to bind double-stranded nucleic acid molecules and biological compartments, biotinylated binding moieties are bound to the streptavidin coated surface of the solid phase.

Yet an other aspect of the invention concerns the use of a solid phase material according to the invention for a target nucleic acid amplification in a sample avoiding the amplification of nucleic acid molecules potentially present in the reagents necessary to amplify said target nucleic acid molecules in said sample.

Thus, by using a solid phase material according to the invention for a target nucleic acid amplification in a sample that avoids the amplification of non-target and/or target nucleic acid molecules potentially present in said reagents, the risk of false positive results due to contaminations can be reduced.

In a preferred use according to the invention, said target nucleic acid amplification is a PCR amplification.

In another preferred use according to the invention, said target nucleic acid amplification is part of a test for microbiological infection of a sample.

Yet an other aspect of the invention concerns a kit comprising the reagents necessary to perform a target nucleic acid amplification and a solid phase material according to the invention.

In a preferred kit according to the invention, said reagents comprise oligonucleotides, nucleotides, enzymes and buffer solutions.

In more preferred kit according to the invention, said oligonucleotides comprise primers and probes and said enzymes comprise DNA-polymerases and uracil-N-glycosylase (UNG).

In another preferred kit according to the invention, said solid phase material comprises pipette tips and vessels.

### Example 1:

### Synthesis of Minor Groove Binder (MGB), peptides and intercalator

### Materials

The rink amide resin and Fmoc-Glu(biotinyl-PEG)-OH were purchased from Novabiochem (Merck Biosciences AG, Läufelfingen, Switzerland). 4-(Fmoc-amino)-1-methylpyrrole-2-carboxylic acid (Fmoc-Amp-OH) and 4-(Fmoc-amino)-1-methyl-1H-imidazole-2-carboxylic acid (Fmoc-Im-OH) were purchased from Fluka and Fmoc-Orn-OH and Fmoc-Nva-OH were purchased from Bachem (Bubendorf, Switzerland). All other amino acids were obtained from Applied Biosystems (Foster City, USA). HBTU and HOBt were from Iris Biotech (Marktredwitz, Germany). Peptide synthesis solvents (DMF, NMP) were obtained from Merck AG (Darmstadt, Germany) and like all the other solvents were of the highest biochemical grade commercially available.

### General synthesis procedure

All the compounds were assembled either manually or on an Applied Biosystems Inc. 433 peptide synthesizer using FastMoc Chemistry. Normal coupling reactions were performed using Fmoc amino acids (1 mmol) activated with HOBt/HBTU and DIPEA for 1 h unless otherwise noted. Fmoc removal was effected by treating the resin for 2x5 min. with 20 % piperidine in DMF. The side chains of trifunctional amino acids were protected as follows: Arg(Pmc), Asn(Trt), Asp(OtBu), Gln(Trt), Glu(OtBu), Lys(Boc), Ser(tBu), Thr(tBu), Trp(Boc), Tyr(tBu).

### 1. Minor Groove Binder:

### MGB 1

### Bi-PEG-Distamycin

### H-Glu(biotinyl-PEG)-Nva-Orn-Nva-Amp-Amp-Amp-Arg-NH2

After conventional anchoring of the first arginine to 0.25 mmol of rink amide resin (0.43 mmol/g) and subsequent removal of its Fmoc group, the resin was successively washed with NMP, isopropanol and NMP. DIPEA (2M in DMF) was added to a solution of Fmoc-Amp-OH (2 mmol) and HOBt/HBTU (1:1; 2mmol). After 2 min. of activation, this mixture was added to the resin. The suspension was shaken for three hours, and the resin was washed thoroughly with DMF and isopropanol. This procedure was repeated three times until all three Amp-residues were incorporated into the sequence. The Fmoc-group was cleaved as described above and the peptide chain was elongated by Nva, Orn, Nva and Glu(biotinyl-PEG) under normal coupling conditions employing double couplings. After cleaving the N-terminal Fmoc-group and washing the resin with DMF, the resin was cleaved with 2.8 ml (per 100 mg of resin) of a mixture containing TFA, triethylsilane and ethandithiol (0.8/0.5/1.5). The resin was filtered off and the peptide was precipitated by 300 ml of cold diisopropylether to the eluate. The precipitate was washed with ether, dried in vacuo and dissolved in acetic acid/water (1/5) for preparative HPLC purification on vydac polygosil.

LC-ESI-MS: m/z = 1410.12 [M+H]⁺; Mᵣ = 1409.59 Da calcd. for C₆₄H₁₀₄N₂₀O₁₄S.

### MGB 2

### Bi-PEG-Distamycin (without Arg)

### H-Glu(biotinyl-PEG)-Nva-Orn-Nva-Amp-Amp-Amp-NH₂

This substance was synthesized according to the procedure as described for MGB 1, except that Fmoc-Arg (Pmc) was not coupled as first amino acid to the resin.

### LC-ESI-MS: m/z = 1253,74 [M+H]⁺; Mᵣ = 1253,51 Da calcd. for C₅₈H₉₂N₁₆O₁₃S

### MGB 3

### Bi-PEG-Imidazol-derivative

### H-Glu(biotinyl-PEG)-Nva-Orn-Nva-Im-Im-Im-Arg-NH₂

This substance was synthesized according to the procedure as described for Bi-PEG-Distamycin, except that Fmoc-Amp-OH was replaced by 4-(Fmoc-amino)-1-methyl-1H-imidazole-2-carboxylic acid (Fmoc-Im-OH).

Cleavage from the resin was achieved with 1.2 ml (per 100 mg of resin) of a mixture containing TFA, water, thioanisol, ethandithiol (1/0.05/0.05/0.1). Workup and purification was done as described above.

LC-ESI-MS: m/z = 1412.86 [M+H]⁺; Mᵣ = 1411.9 Da calcd. for C₆₁H₁₀₁N₂₃O₁₄S.

### MGB 4

### Bi-PEG-Imidazol- derivative (short)

### H-Glu(biotinyl-PEG)-Im-Im-Im-Arg-NH₂

This substance was synthesized according to the procedure as described for MGB 3, except that Fmoc-Glu(biotinyl-PEG)-OH was directly coupled to Im.

LC-ESI-MS: m/z = 1100,5 [M+H]⁺; Mᵣ = 1100.20 Da calcd. for C₄₆H₇₃N₁₉O₁₁S

### 2. Peptides:

Synthesis was carried out according to the general procedure described above. Cleavage from the resin was achieved as described for the imidazole distamycin compound and the substances were purified by preparative HPLC.

### Peptide 1

H-Glu(biotinyl-PEG)-QGRVEVLYRGSWGTVA-NH₂
LC-ESI-MS: m/z = 1168.06 [M+2H]²⁺; Mᵣ = 2334.1 Da. calcd. for C₁₀₄H₁₆₈N₃₀O₂₉S

### Peptide 2

H-Glu(biotinyl-PEG)-IGAVLKVLTTGLPALISWIKRKRQQ-NH₂
LC-ESI-MS: m/z = 1116.58 [M+3H]³⁺; Mᵣ = 3346.7 Da. calcd. for C₁₅₄H₂₆₉N₄₃O₃₇S.

### Peptide 3

Bi-ZU-KKNKRNTNRRPQDVKFPGGGQIVGGVYLLPRRGPRLGVRA-NH₂
LC-ESI-MS: m/z = 696.6 [M+7H]⁷⁺; Mᵣ = 4869.2 Da.

### Peptide 4

Bi-XUZU-KKNKRNTNRRPQDVKFPGGGQIVGGVYLLPRRGPRLGVRATRKTS-NH₂
Mᵣ = 5639.8 Da

### Peptide 5

Bi-XUZU-PWPLYGNEGLGWAGWLLSPRGSRPSWGPTDPRRRSR-NH₂
Mᵣ = 4728.8 Da

### Peptide 6

Bi-XUUUU-QPGPPSEKAWQPGWT-NH₂
Mᵣ = 2303.6 Da

### Peptide 7

BXUZU-GGGGDDLGANDELISFKDEGEQEEK(Amid)
Mᵣ = 3219,44 g/mol

### Peptide 8

***H-Glu(biotinyl-PEG)-(Arg-Gly)***_{***5***}***-NH***_{***2***}
LC-ESI-MS: m/z = 411.39 [M+4H]⁴⁺; Mᵣ = 1640.9 Da calcd. for C₆₅H₁₂₁N₃₁O₁₇S.

### Peptide 9

***H-Glu(biotinyl-PEG)-(Arg-Gly)***_{***15***}***-NH***_{***2***}
LC-ESI-MS: m/z = 755.8 [M+5H]⁵⁺; Mᵣ = 3773.3 Da calcd. for C₁₄₅H₂₇₁N₈₁O₃₇S.

### Peptide 10

***H-Glu(biotinyl-PEG)-(Lys-Gly)***_{***5***}***-NH***_{***2***}
LC-ESI-MS: m/z = 501.51 [M+3H]³⁺; Mᵣ = 1501.4 Da calcd. for C₆₅H₁₂₁N₂₁O₁₇S.

### Peptide 11

***H-Glu(biotinyl-PEG)-(Lys-Gly)***_{***15***}***-NH***_{***2***}
LC-ESI-MS: m/z = 839.43 [M+4H]⁴⁺; Mᵣ = 3354.6 Da calcd. for C₁₄₅H₂₇₁N₅₁O₃₇S.

### Peptide 12

***H-Glu(biotinyl-PEG)-(Arg)***_{***20***}***-NH***_{***2***}
LC-ESI-MS: m/z = 617.61 [M+6H]⁶⁺; Mᵣ = 3699.5 Da calcd. for C₁₄₅H₂₈₆N₈₆O₂₇S

### Abbreviations

The nomenclature of amino acids and peptides is in accordance with the proposals of the IUPAC-IBU Commission on Biochemical Nomenclature (Europ. J. Biochem. 138 (1984) 9-37)
- ABI: Applied Biosystems

- Amp: Aminomethylpyrrole-2-carboxylic acid
- Boc: tert.-Butyloxycarbonyl
- Da: Dalton
- DIPEA: Diisopropylethylamine
- DMF: Dimethylformamide
- ESI-MS: Electrospray Ionisation Mass Spectrometry
- Fmoc: Fluorenyl-9-methyloxycarbonyl
- HBTU: 2(1H-Benzotriazol-1yl)-1,1,3,3-tertramethyluroniumhexafluorophosphate
- HOBt: 1-Hydroxybenzotriazole
- HPLC: High Performance Liquid Chromatography
- Im: Aminomethyl-1H-imidazole-2-carboxylic acid
- MS: mass spectrometry
- NMP: N-Methylpyrrolidin
- OtBu: O-tert.-Butyl
- PEG: Polyethylenglycol
- Pmc: 2,2,5,7,8-Pentylmethylchroman-6-sulfonyl
- TFA: Trifluoroacetic acid
- Trt: Triphenylmethyl (Trityl)

### 3. Intercalator:

Acriflavine-Biotin Conjugate: 2, 7-Bis(amino)-9-(biotinylamidoethylamino) acridine

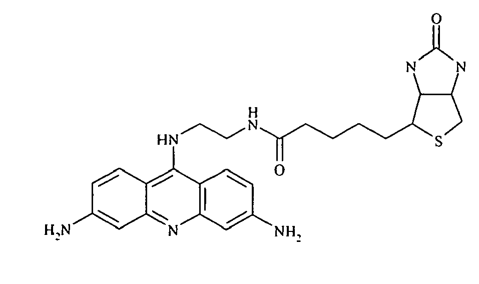

Toronto Reasearch Chemicals, North York, Canada; Cat.Nr.: A191100; Mᵣ = 493.64 Da (C₂₅H₃₁N₇O₂S)

### Example 2:

Several biotinylated ds DNA-binder (Example 1) were immobilized onto Streptavidin coated Dynabeads® M-280 (Dynal Biotech S.A., Prod. No. 112.06, Oslo, Norway ; binding capacity for biotin: 650 pmol/mg ; SA-magnetic beads throughout these examples) according to the following procedure:

200µl (2mg) of resuspended Dynabeads® M-280 were transferred to a PP tube. The tube was placed on a magnet for 1-2 min. After removing the supernatant, the beads were washed twice with 500 µl of washing&binding buffer (5 mM Tris-HCl pH 7.5; 0,5 mM EDTA; 1.0 M NaCl). Thereafter, 200 µl of washing&binding buffer and 2µl (2000pmol) of biotinylated ds DNA-binder (c = 1000pmol/µl) were added and incubated for 60 min at room temperature on an Eppendorf thermomixer comfort. The supernatant was removed from the derivatized beads by supplying a magnet, then the beads were washed 5 times with 500 µl of washing&binding buffer. The derivatized beads were stored in washing&binding buffer until usage (final concentration: 1mg derivatized beads / 100µl washing&binding buffer).

### Example 3:

The minor groove binder MGB1, MGB3 and the peptide 3 (Example 1) were immobilized onto Streptavidin coated Dynabeads® M-280 (Dynal Prod. No. 112.06, Oslo, Norway; binding capacity for biotin: 650 pmol/mg; SA-magnetic beads throughout these examples) according to the following procedure:

200 µl of resuspended Dynabeads® M-280 were transferred to a PP tube. The tube was placed on a magnet for 1-2 min. After removing the supernatant, the beads were washed twice with 200 µl of washing&binding buffer (5 mM Tris-HCl pH 7.5; 0,5 mM EDTA; 1.0 M NaCl). Thereafter, 200 µl of washing&binding buffer and 20 µl (20 µmol) of biotinylated ds DNA-binder were added and incubated for 60 min at room temperature on a Eppendorf thermomixer. The supernatant was removed from the derivatized beads by supplying a magnet, then the beads were washed 3-4 times with 200 µl of washing&binding buffer. The derivatized beads were stored in washing&binding buffer until usage (final concentration: 1 mg derivatized beads / 100 µl washing&binding buffer).

### Example 4:

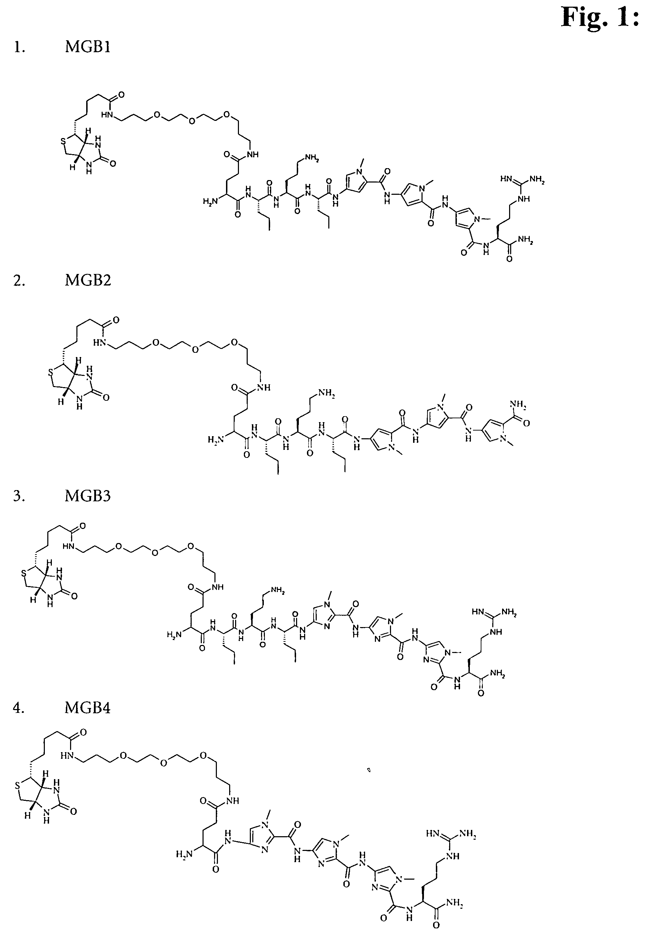

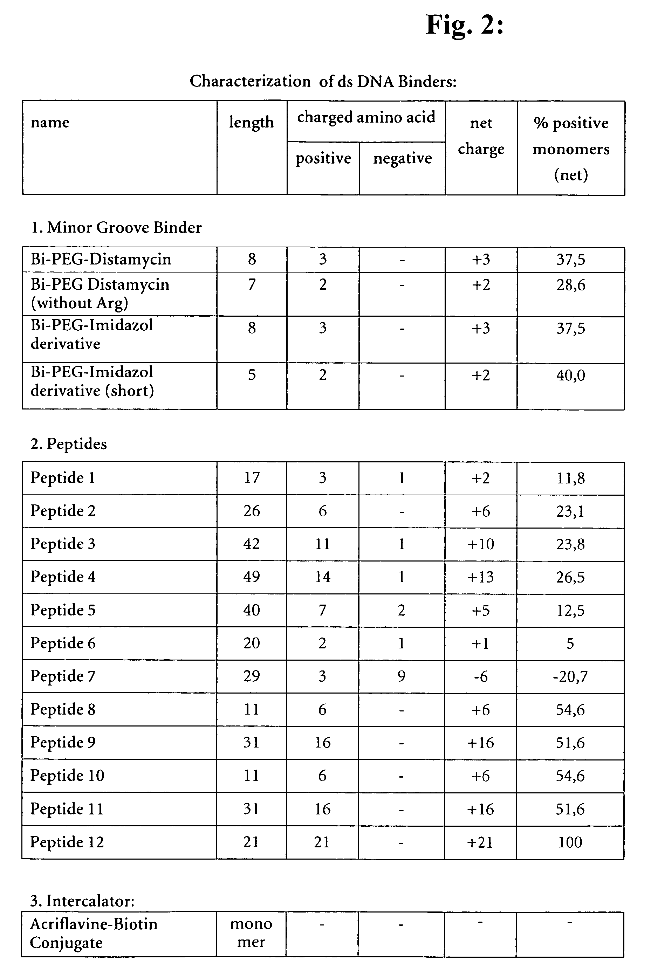

The minor groove binder MGB1, MGB3 and the peptide 3 (Figure 1 and Figure 2) were immobilized onto Streptavidin-coated PCR tubes (Roche Cat. No. 1741772, Roche Diagnostics GmbH, Mannheim; binding capacity for biotin: 61,5 pmol/200 µl tube) according to the following procedure:

The tubes were washed twice with buffer (50 mM Tris-HCl pH 8.1; LC Sepsis Kit, Id.nr.04493613, Roche Diagnostics GmbH, Germany), then 195 µl of buffer and 5 µl of biotinylated ds-NA binder (in 10 mM Tris, pH 8.0 ; c = 1000 pmol/µl) were added. After incubating for 20 min at 37 °C (water bath) the supernatant was carefully removed with a pipet, and the tubes were washed with buffer 3-4 times.

### Example 5:

In this experiment the decontamination from bacterial DNA (Staphylococcus epidermidis) with several minor groove binder/SA-magnetic bead complexes was performed.

For each experiment 50µl (0,5 mg) of MGB/SA-magnetic bead complex (see example 2) were transferred in a sterile and siliconized tube, washed with 250µl buffer (10 mM Tris-HCl pH 8.0) about four times and the supernatant was removed completely. Afterwards, 40 µl of a Staphylococcus epidermidis DNA dilution (DNA standard from LC Sepsis Test, Roche Identnr.: 04493613) were added to each tube. During incubation at room temperature the tubes were mixed for 60 min. The supernatants were analyzed by PCR on a Light Cycler 1.2. The standard curve was obtained by a 1:10 dilution series of the bacterial DNA (Staphyloccocus epidermidis stock solution ; c = 10⁵ cp/10µl) in 50 mM Tris-HCl buffer pH 8.1.

The quantification reactions were run on the Light Cycler Version 1.2 using the Light Cycler Staphylococcus Kit M^{grade} (Roche Cat.No. 3 376 419, Roche Diagnostics GmbH, Mannheim) with the following cycling conditions: denaturation at 95 °C for 10 min, then 45 cycles comprising of a profile of 50 °C for 15 s with fluorescence measurement, 72 °C for 10 s and 95 °C for 10 s. Following cycling a melt analysis was done immediately by rapid cooling to 40 °C and then increasing the temperature to 95 °C with a ramp rate of 0,1 °C/min using continous fluorescence measurement.

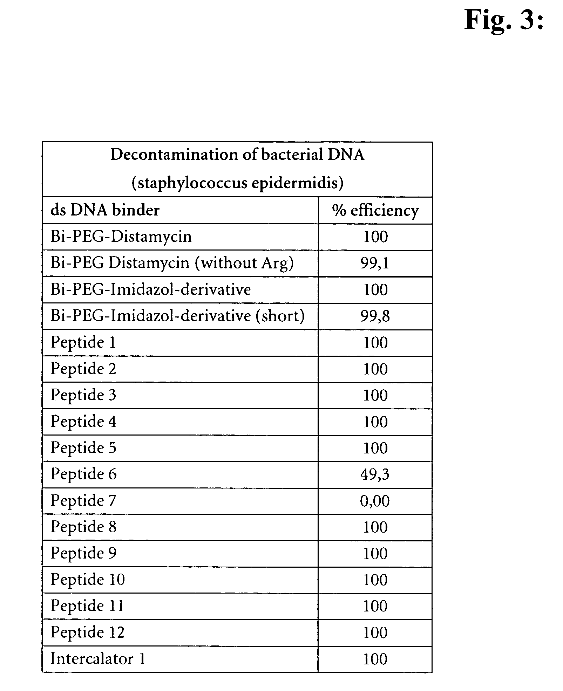

The results of this experiment are summarized in Figure 3. In the experiment 1,7 x 10⁴ cp were decontaminated. As a conclusion the decontamination efficiency is in the range of 10³-10⁴ for all binders except peptides 6 and 7.

### Example 6:

The decontamination efficiency of several ds DNA-binder molecules was estimated using the Light Cycler Control Kit DNA (Roche Cat.No. 2158833, Roche Diagnostics GmbH, Mannheim), Light Cyler DNA Master Hybridization Probes (Roche Cat.No. 2 015 102, Roche Diagnostics GmbH, Mannheim) and the LightCycler 1.2 apparatus (Roche Cat.No 2 011 468; Software Version LC 3.5).

For each decontamination experiment 60µl of a hg DNA (template for control reaction from Light Cycler Control Kit DNA (110bp fragment of β-globin gene)) containing solution was decontaminated with 0,5mg of ds DNA binder/SA-magnetic bead complex (same preparation as in example 2). The standard curve was obtained by a 1:10 dilution series of the hg DNA template from kit in 10mM Tris pH 8.0.

After incubation for 60 min at room temperature on an Eppendorf thermomixer comfort the supernatant was analyzed by PCR according to the kit instruction manual.

Quantifications were run on the Light Cycler apparatus and the assay was performed according to the procedure of the kit instruction manual and using the following cycling conditions: denaturation at 95 °C for 30s, then 45 cycles comprising a profile of 55 °C for 10 s with fluorescence measurement, 72 °C for 5 s and 95 °C for 0 s and final cooling to 40 °C with a ramp rate of 20 °C/s. The standard curve was evaluated with a dilution series of hg DNA from the kit.

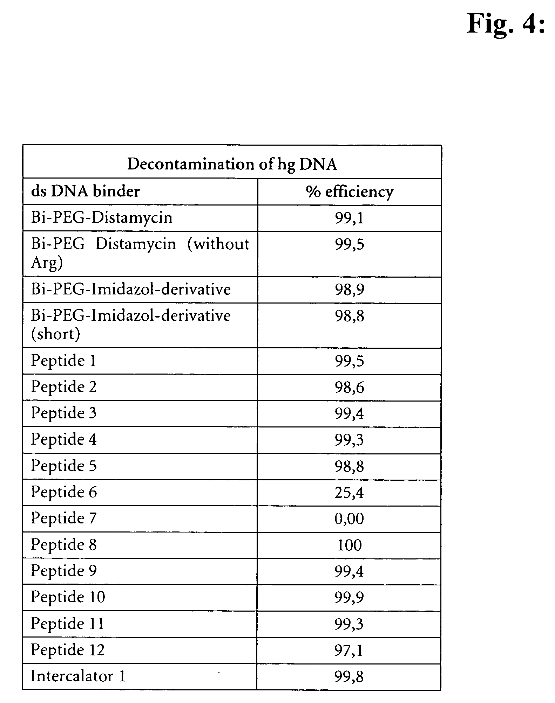

The results of this experiment are summarized in Figure 4. In the experiment 1,2 x 10⁴ cp were decontaminated. As a conclusion the decontamination efficiency is in the range of 10²-10⁴ for all binders except peptides 6 and 7.

### Example 7:

The decontamination efficiency of the three ds-NA (MGB1, MGB3 and the peptide 3) binders was estimated using the Parvovirus B19 Quantification Kit (Roche Cat.No. 3 246 809, Roche Diagnostics GmbH, Germany) and the LightCycler 1.2 apparatus (Roche Cat.No. 2011468, Roche Diagnostics GmbH, Germany; Software Version LC 3.5).

For each decontamination experiment 10 µl (0.1 mg) of ds-NA binder / SA-magnetic bead complex from example 3 were transferred to a PP tube and washed twice with 10 mM Tris, pH 8.0. After removal of supernatant, 20 µl of a plasmid DNA (Parvovirus DNA standard from the Parvovirus B19 Quantification Kit Roche Cat.No. 3 246 809, Roche Diagnostics GmbH, Germany) containing solution were added. After incubation for 60 min at room temperature on a mixer (Thermomixer Comfort, Cat.no. 5350000.013, Eppendorf, Germany) the supernatant was analyzed by PCR according to the kit instruction manual.

Quantifications were run on the Light Cycler apparatus and the assay was performed according to the procedure of the kit instruction manual without the internal control and using the following cycling conditions: denaturation at 95 °C for 10 min, then 45 cycles comprising a profile of 60 °C for 15 s with fluorescence measurement, 72 °C for 10 s and 95 °C for 10 s and final cooling to 40 °C with a ramp rate of 20 °C/s. The standard curve was evalutated with DNA standards from the kit.

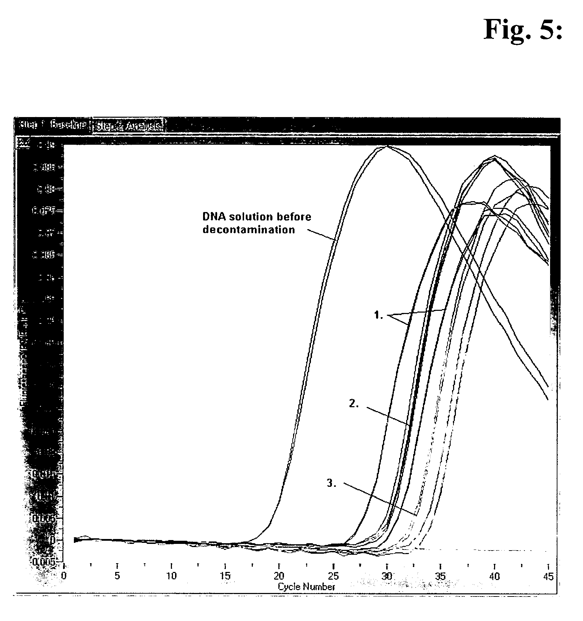

The amplification curves of these decontamination experiments are summarized in Figure 5. As a conclusion the following decontamination efficiencies were obtained: 4 x 10² (for MGB 1), 2,6 x 10² (for MGB 3) and 1,6 x 10³ (for peptide 3).

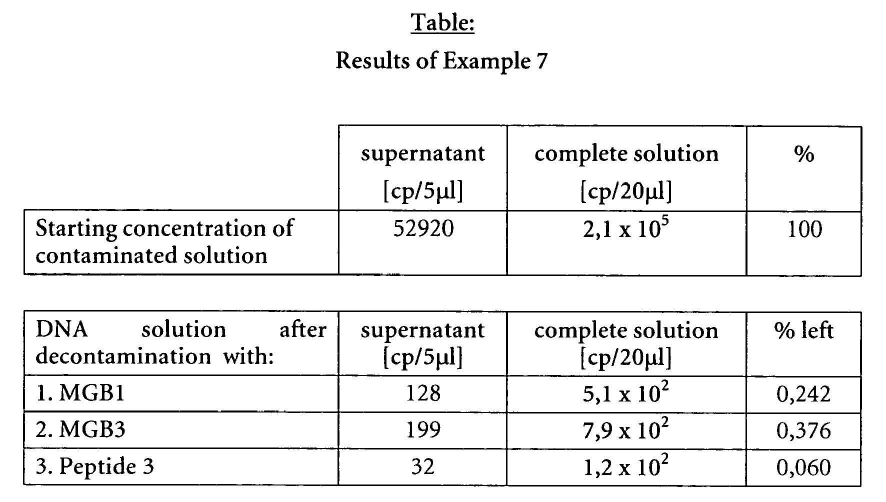

### Example 8:

The following experiment was performed in order to evaluate the selectivity of the three different ds-NA binders (MGB1, MGB3 and the peptide 3) with respect to double-stranded and single-stranded oligonucleotides in a time dependent manner.

Two single-stranded oligonucleotide solutions (5'-CCC ATC CCC AAA AAC ACA AAC CAC A PO4-3' ; c = 14,189 OD/ml [49,9µM] in 10 mM Tris ph 8.0), one with and one without plasmid DNA (Parvovirus DNA standard from the Light Cycler Parvovirus B19 Quantification Kit, Roche Cat.No. 3 246 809, Roche Diagnostics GmbH, Mannheim) were both decontaminated with ds-NA binder/SA-magnetic bead complexes (from example 3) in a time dependent manner. The oligonucleotide concentration in the supernatant was determined after 1, 6 and 48 h by UV-adsorption (UVIKON 931 spectrophotometer, Kontron, Germany) and additionally, in case of the solution containing plasmid DNA, the decontamination efficiency was evaluated by PCR as described in the preceding examples. For sake of comparison, a solution of just the plasmid DNA in buffer (TBE buffer: 10 mM Tris-HCl pH 8.0) was decontaminated and quantified by PCR as well.

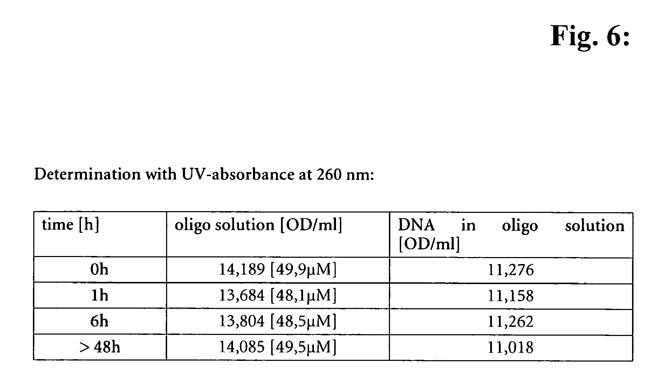

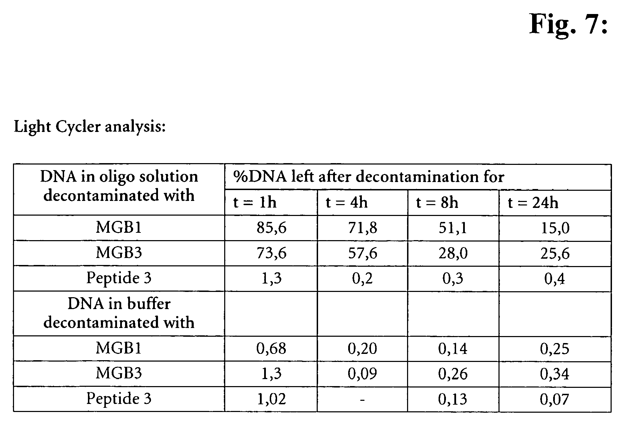

The Parvovirus B19 plasmid standard DNA (c = 5 x 10⁵ cp/µl) was added to one of the single stranded oligonucleotide solutions (ratio: 1: 4 (^{v}/ᵥ)). For each experiment of Figure 6 and 7, 100 µl (1 mg) of Bi-PEG-Distamycin/SA-magnetic bead complex in washing&binding buffer were transferred into a siliconized and sterile PP tube, washed twice with 10 mM Tris, pH 8.0 and the supernatant was removed completely. Thereafter, 200 µl of both oligonucleotide solutions were added to two different tubes containing the beads and the absorbance of the oligo solutions was determined (λ = 260 nm) after 1, 6 and 48 h in order to determine the concentration in the supernatant. Additionally, the plasmid DNA containing solutions with and without single stranded oligonucleotides were analyzed by PCR on a Light Cycler 1.2 as described in the preceding experiments using all three ds-NA binders (Figure 7).

The results of this experiment are summarized in Figure 6 and 7. From the adsorbance experiment (Figure 6) it can be seen that the ds DNA binding selectivity of the Bi-PEG-Distamycin/SA-magnetic bead complex in the presence of ss oligonucleotide is provided. Even in the presence of a large amount of single-stranded oligonucleotides, the ds DNA molecules are efficiently removed from the sample. The LightCycler quantification (Figure 7) demonstrates that the time-dependent selective binding of ds DNA in the presence of ss oligonucleotide is best, if the biotinylated peptide 3 is used as ds-NA binder.

### Example 9:

In this experiment the decontamination from bacterial DNA (streptoccocus pneumonia DNA from LC Sepsis Kit, Id.nr.04493613, Roche Diagnostics GmbH, Germany) with the Bi-PEG-Distamycin/SA-magnetic bead complex was performed and subsequently, the bound DNA was eluted from the beads.

For each experiment 10 µl (0.1 mg) of Bi-PEG-Distamycin/SA-magnetic bead complex (from example 3) were transferred in a sterile and siliconized tube, washed with buffer (50 mM Tris-HCl pH 8.1) twice and resuspended in 20 µl of the same buffer. The standard curve was obtained by a 1:10 dilution series of the bacterial DNA (Streptoccocus pneumonia stock solution ; c = 10⁶ cp/µl) in 50 mM Tris-HCl buffer pH 8.1.

Afterwards, 20 µl of streptoccocus pneumonia DNA dilution were added to each tube. During incubation at room temperature the tubes were mixed for 60 min. The supernatants were analyzed by PCR on a Light Cycler 1.2. The quantification reactions were run on the Light Cycler Version 1.2 (Cat.No. 2011468, Roche Diagnostics GmbH, Germany; Software Version LC 3.5) using the Light Cycler Sepsis Kit (Id.nr.04493613, Roche Diagnostics GmbH, Germany) with the following cycling conditions: denaturation at 95 °C for 10 min, then 45 cycles comprising of a profile of 60 °C for 25 s with fluorescence measurement, 72 °C for 40 s and 95 °C for 20 s. Following cycling a melt analysis was done immediately by rapid cooling to 40 °C and then increasing the temperature to 95 °C with a ramp rate of 0,1 °C/min using continous fluorescence measurement.

After removal of the supernatant, each tube (containing Bi-PEG-Distamycin/SA-magnetic bead complex) was washed twice with PCR-water (from Sepsis Kit). Then 40 µl of 10 mM NaOH were added and the tubes were incubated for 60 min at room temperature on a mixer (Thermomixer Comfort, Cat.no. 5350000.013, Eppendorf, Germany). The supernatants containing the DNA eluted from the beads were analyzed by PCR.

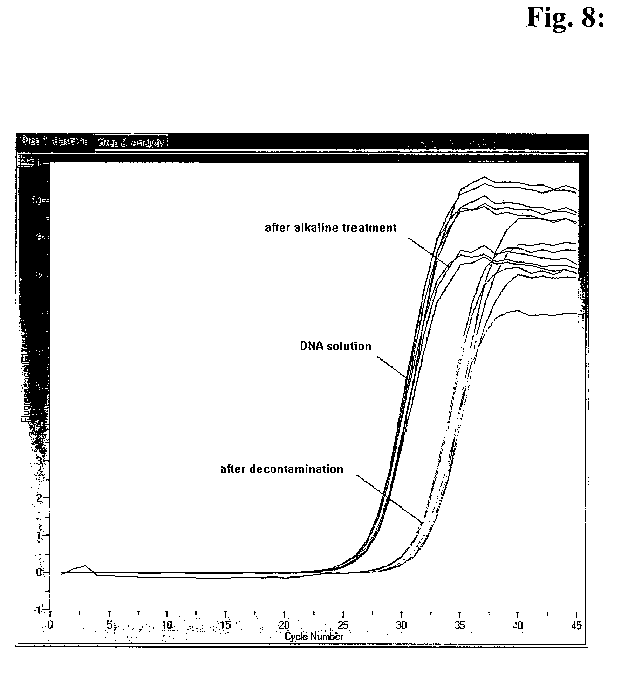

The amplification curves of this experiment are summarized in Figure 8, demonstrating the decontamination efficiency towards bacterial DNA. After decontamination, the supernatant contained 17 cp/µl, whereas after subsequent elution of bound molecules from the beads the supernatant contained as much as 340 cp/µl.

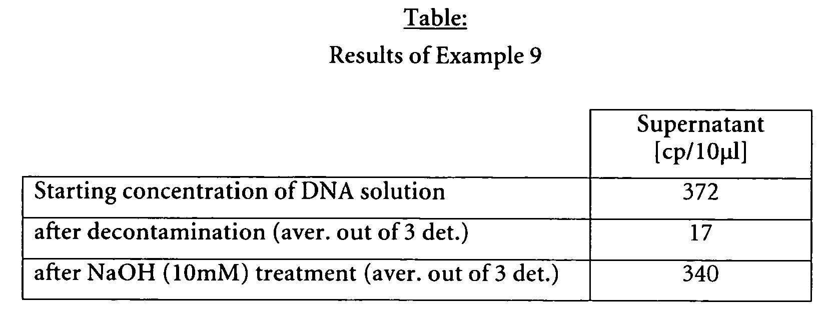

### Example 10:

In this experiment the decontamination of bacterial DNA (streptoccocus pneumonia DNA from LC Sepsis Kit, Id.nr.04493613, Roche Diagnostics GmbH, Germany) in a Bi-PEG-Distamycin/SA-coated tube was performed and subsequently, the bound DNA was eluted from the tubes.

A solution of streptoccocus pneumonia DNA (Streptoccocus pneumonia stock solution; c = 10⁶ cp/µl) in buffer (TBE buffer: 50 mM Tris-HCl pH 8.1) was prepared (ratio 1:1 (^{v}/ᵥ)). The standard curve was obtained by a 1:10 dilution series of the bacterial DNA in 50 mM Tris-HCl buffer pH 8.1.

40 µl of the prepared solution were added to each tube (Bi-PEG-Distamycin/SA-coated tube complex from example 4) and incubated at room temperature for 60 min on a mixer (Thermomixer Comfort, Cat.no. 5350000.013, Eppendorf, Germany). The supernatants were analyzed by PCR on a Light Cycler 1.2 (Cat.No. 2011468, Roche Diagnostics GmbH, Germany; Software Version LC 3.5) using the Light Cycler Sepsis Kit, whereas the quantification reactions were performed with the following cycling conditions: denaturation at 95 °C for 10 min, then 45 cycles comprising of a profile of 60 °C for 25 s with fluorescence measurement, 72 °C for 40 s and 95 °C for 20 s. Following the cycling a melt analysis was performed immediately by rapid cooling to 40 °C and then increasing the temperature to 95 °C with a ramp rate of 0,1 °C/min using continous fluorescence measurement.

After removal of the supernatant, each tube was washed twice with PCR-water (from Sepsis Kit). Then 40 µl of 10 mM NaOH were added and the tubes were incubated for 60 min at room temperature on a mixer. The supernatants containing the DNA eluted from the tubes were analyzed by PCR accordingly.

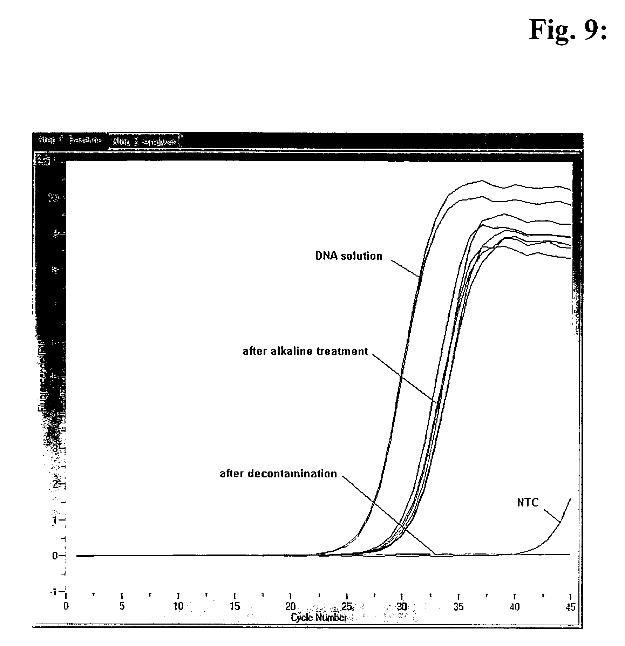

The amplification curves of this experiment are summarized in Figure 9, demonstrating the decontamination efficiency of the tubes towards bacterial DNA. After decontamination, the supernatant contained no detectable molecules at all, whereas after subsequent elution of bound molecules from the tubes the supernatant contained as much as 38 cp/µl.

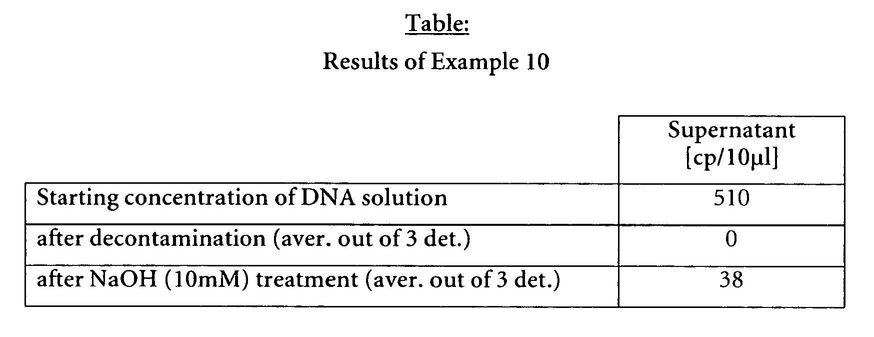

### Example 11:

In this experiment the decontamination from bacteria (Staphylococcus epidermidis) with several bacteria binder/SA-magnetic bead complexes was performed.

For each experiment 50 µl (0,5 mg) of bacteria binder/SA-magnetic bead complex (see example 2 for the immobilization of biotinylated binder on Streptavidin coated Dynabeads®) were transferred in a sterile and siliconized tube, washed with 250 µl buffer (10 mM Tris-HCl pH 8.0) about four times and the supernatant was removed completely. Afterwards, 40 µl of a Staphylococcus epidermidis bacteria dilution were added to each tube. During incubation at room temperature the tubes were mixed for 60 min. The supernatants were analyzed by PCR on a Light Cycler 1.2 (Roche Diagnostics GmbH, Germany; Software Version LC 3.5). The standard curve was obtained by a 1:10 dilution series of the bacterial stock solution (Staphyloccocus epidermidis stock solution ; c = 9·10³ DNA equivalents copies/10 µl) in 10 mM Tris-HCl buffer pH 8.0.

The quantification reactions were run on the Light Cycler Version 1.2 using the Light Cycler Staphylococcus Kit M^{grade} (Roche Cat.No. 3 376 419, Roche Diagnostics GmbH, Germany) with the following conditions: denaturation at 95 °C for 10 min, then 45 amplification cycles each with the following profil: 50 °C for 15 s with a fluorescence measurement, 72 °C for 10 s and 95 °C for 10 s. After the amplification reaction an immediate melt analysis was performed by rapid cooling to 40 °C and then increasing the temperature to 95 °C with a ramp rate of 0,1 °C/min using continous fluorescence measurements.

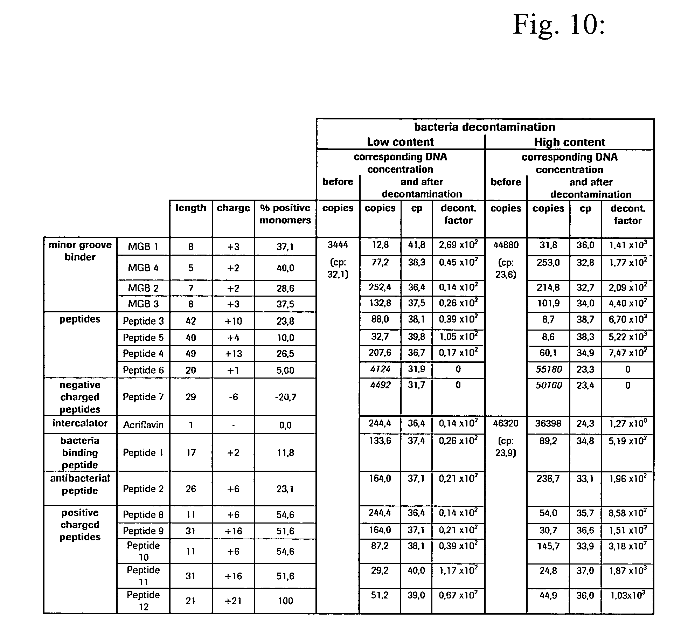

The results of this experiment are summarized in Figure 10. In the experiments about 3,4·10³ and 4,5·10⁴ copies of bacteria were removed. As a conclusion, intact bacteria can be depleted with decontamination efficiencies up to the range of 10³, whereas minor groove binders and positively charged peptides worked best, and negatively charged peptides did not function at all.

### List of References

Abramson, R.D. and Myers, T.W. Current Opinion in Biotechnology 4 (1993) 41-47
Abravaya, K. et al, Nucleic Acid Ampl. Technol. Chapter 9 (1997) 125-133
Aslam & Dent: Bioconjugation (2000, Macmillian Reference, London, GB
Ausubel et al.: Current Protocols in Molecular Biology (1987) J. Wiley and Sons, NY
Barany, F., Proc. Natl. Acad. Sci. USA 88 (1991) 189-193
Barany, F., PCR Methods and Applic. 1 (1991) 5-16
Boger, D.L. et al., J. Am. Chem. Soc. 123 (2001) 5878-5891
Brana, M.F. et al, Curr. Pharm. Design 7 (2001) 1745-1780
Corless, C.E. et al, J. Clin. Microbiol. 38 (2000) 1747-1752
Demeunynck et al (Eds.): DNA and RNA binders (Vol. 1 & 2, 2003, Wiley-VCH, Weinheim
Di Pietro, S.M. et al., Biochemistry 42 (2003) 6218-6227
Doré, K. et al., JACS 126 (2004) 4240-4244
EP 0 281 390
EP 0 439 182
EP 0 476 014
EP 0 585 660
EP 0 792 376
Fish, E.L. et al, Biochemistry 27 (1988) 6026-6032
Fox, J.C. et al, J. Virol. Meth. 33 (1991) 375-3823
Guatelli, J.C. et al., Proc. Natl. Acad. Sci. USA 87 (1990) 1874-1878
Hilali, F. et al, Mol. Biotechnol. 7 (1997) 207-216
Hoheisel, J. D., TIBTECH 15 (1997) 465-469
IUPAC-IBU Commission on Biochemical Nomenclature, Europ. J. Biochem. 138 (1984) 9-37
Kessler (Ed.): Nonradioactive labeling and detection of biomolecules, 1992, Springer Verlag, Heidelberg
Klaschik, S. et al, Mol. Biotechnol. 22 (2002) 231-242
Koepsel, R.R. and Russell, A.J., Biomacromolecules 4 (2003) 850-855
Kwoh, D.Y. et al., Proc. Natl. Acad. Sci. USA 86 (1989) 1173-1177
Li, M. et al Bioorg. Med. Chem. Letters 12 (2003) 4351-4354
Mantero, G. et al, Clin. Chem. 37 (1991) 422-429
Pauluhn, J. et al., Ber. Bunsenges. Phys. Chem. 82 (1978) 1265-1278
Prince, A.M. and Andrus, L., Biotechniques 12 (1992) 358-360
Record, M.T. et al., J. Mol. Biol. 107 (1976) 145-158
Sambrook, J. et al.: Molecular Cloning: A Laboratory Manual (2nd ed., Cold Spring Harbour Laboratory Press, Cold Spring Harbour, NY
Stewart, K.D. et al., J. Phys. Org. Chem. 5 (1992) 461-466
Stryer, Biochemistry, 4th edition (1995) W H Freeman & Co, NV, USA
Tijssen, P.: Practice and theory of enzyme immunoassays (1985, Elsevier, Amsterdam, Netherlands
US 4,683,195
US 5,002,867
US 5,130,238
US 5,143,854
US 5,202,231
US 5,210,015
US 5,487,972
US 5,545,522
US 5,716,785
US 5,804,375
US 5,891,636
US 6,022,963
US 6,103,476
US 6,156,501
US 6,174,670
US 6,291,170
US 2002/0006617
US 2001/0026921
US 2002/0095073
US 2003/0130499
Vogelstein, B., and Gillespie, D., Proc. Natl. Acad. Sci. USA 76 (1979) 615-619
Whelen, A.C. and Persing, D.H., Annu. Rev. Microbiol. 50 (1996) 349-373
WO 00/69872
WO 01/09370
WO 01/94573
WO 02/06117
WO 02/48164
WO 03/33698
WO 04/01418
WO 89/10977
WO 89/11548
WO 90/01069
WO 90/15070
WO 91/19003
WO 92/0880
WO 92/10092
WO 93/17126
WO 2002/060539
WO 2002/074993
WO 2002/82078
Wu, D.Y. and Wallace, R.B., Genomics 4 (1989) 560-569
Zimmer, G. et al., Prog. Biophys. Molec. Biol. 47 (1986) 37-112

## Claims

1. A method for the detection of biological molecules in a sample that avoids the detection of biological molecules potentially present in the reagents necessary to detect the biological molecules in the sample, comprising the steps of
a) providing a sample potentially comprising said biological molecule,
b) providing binding moieties coupled to the surface of a solid phase,
c) providing reagents necessary to detect the biological molecules,
d) adding said reagents to said sample,
e) detecting the biological molecules in the sample,
wherein in step c) or in step d) or in step c) and d) said reagents are in physical contact with said binding moieties under conditions, whereby said biological molecules potentially present in said reagents bind to said binding moieties coupled to said surface of a solid phase.

2. A method according to claim 1, wherein said surface of a solid phase is the inner surface of a vessel or of a pipette tip.

3. A method according to claim 1, wherein said surface of a solid phase is the surface of beads or the surface of a porous material.

4. A method according to claims 1 to 3, wherein the binding affinity of said binding moieties realize a reduction of the biological molecule content in the reagents by at least a factor of 10², preferably by at least a factor of 10³.

5. A method according to claims 1 to 4, wherein said binding moieties are moieties capable of binding double-stranded nucleic acid molecules.

6. A method according to claims 1 to 4, wherein said binding moieties are moieties capable of binding biological compartments.

7. A method according to claims 1 to 4, wherein said binding moieties are moieties capable of binding double-stranded nucleic acid molecules and biological compartments.

8. A method to amplify a target nucleic acid molecule comprising the steps of
a) providing a sample potentially comprising target nucleic acid molecules,
b) providing nucleic acid binding moieties coupled to the surface of a solid phase,
c) providing reagents necessary to amplify said target nucleic acid molecules,
d) adding said reagents to said sample,
e) amplifying said target nucleic acid molecules in said sample,
wherein in step c) or in step d) or in step c) and d) said reagents are in physical contact with said nucleic acid binding moieties under conditions, whereby said nucleic acid molecules potentially present in said reagents bind to said nucleic acid binding moieties coupled to said surface of a solid phase.

9. A method according to claim 8, wherein said surface of said solid phase is the inner surface of a vessel or of a pipette tip.

10. A method according to claim 8, wherein said surface of said solid phase is the surface of beads or the surface of a porous material.

11. A method according to claims 8 to 10, wherein said nucleic acid binding moieties are double-stranded nucleic acid binding moieties.

12. A method according to claim 11, wherein said double-stranded nucleic acid binding moieties are polycationic entities, minor groove binders, intercalators or anti double-stranded nucleic acid antibodies.

13. A method according to claims 11 to 12, wherein biotinylated double-stranded nucleic acid binding moieties are bound to the streptavidin coated surface of the solid phase.

14. A method according to claims 8 to 13, wherein the amplification of said target nucleic acid molecule is a nucleic acid amplification in a test for microbiological infection of a sample.

15. A solid phase material, whereas double-stranded nucleic acid binding moieties are coupled to the surface of said solid phase material and whereas said solid phase material is a vessel, a bead or a pipette tip.

16. A solid phase material according to claim 15, whereas said double-stranded nucleic acid binding moieties are polycationic entities, minor groove binders, intercalators or anti double-stranded nucleic acid antibodies.

17. A solid phase material, whereas polycationic entities or intercalators are coupled to the surface of said solid phase material as double-stranded nucleic acid binding moieties.

18. A solid phase material, whereas binding moieties are coupled to the surface of said solid phase material that are able to bind biological compartments and whereas said solid phase material is a vessel or a pipette tip.

19. A solid phase material according to claim 18, whereas said binding moieties are minor groove binders, antibodies, dyes, amphiphilic entities or polycationic entities.

20. A solid phase material, whereas dyes, amphiphilic entities, polycationic entities or minor groove binders are coupled to the surface of said solid phase material as binding moieties for biological compartments.

21. A solid phase material, whereas binding moieties are coupled to the surface of said solid phase material that are able to bind double-stranded nucleic acid molecules and biological compartments.

22. A solid phase material according to claim 21, whereas said solid phase material is a bead, a porous material, a vessel or a pipette tip.

23. A solid phase material according to claims 21 to 22, whereas two or more kinds of binding moieties are coupled to the surface of said solid phase material.

24. A solid phase material according to claim 23, whereas said two or more binding moieties are choosen from the group consisting of minor groove binders, intercalators, antibodies, dyes, amphiphilic entities or polycationic entities.

25. Use of a solid phase material according to claims 15 to 24 for a target nucleic acid amplification in a sample avoiding the amplification of nucleic acid molecules potentially present in the reagents necessary to amplify said target nucleic acid molecules in said sample.

26. Use according to claim 25, whereas said target nucleic acid amplification is part of a test for microbiological infection of a sample.

27. A kit comprising the reagents necessary to perform a target nucleic acid amplification and a solid phase material according to claims 15 to 24.
